(19) Europäisches Patentamt
European Patent Office
Office européen des brevets

(11) Publication number: **0 061 907**
**B1**

(12) # EUROPEAN PATENT SPECIFICATION

(45) Date of publication of the patent specification: **01.08.84**

(51) Int. Cl.³: **C 07 C 91/16,** C 07 D 307/81, A 61 K 31/135, A 61 K 31/34

(21) Application number: **82301594.6**

(22) Date of filing: **26.03.82**

(54) **Secondary amines, their preparation, pharmaceutical compositions containing them and their use.**

(30) Priority: **31.03.81 GB 8110036**
**30.12.81 GB 8139023**

(43) Date of publication of application:
**06.10.82 Bulletin 82/40**

(45) Publication of the grant of the patent:
**01.08.84 Bulletin 84/31**

(84) Designated Contracting States:
**BE CH DE FR GB IT LI NL SE**

(56) References cited:
**EP - A - 0 021 636**
**DD - A - 144 761**
**US - A - 3 517 000**
**US - A - 3 859 279**

(73) Proprietor: **BEECHAM GROUP PLC, Beecham House Great West Road, Brentford Middlesex (GB)**

(72) Inventor: **Smith, David Glynn, 36 Shrewsbury Road, Redhill Surrey (GB)**

(74) Representative: **Russell, Brian John et al, European Patent Attorney Beecham Pharmaceuticals Great Burgh Yew Tree Bottom Road, Epsom Surrey, KT18 5XQ (GB)**

EP 0 061 907 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to derivatives of ethanolamine, to processes for their production and to their use in the treatment of obesity, hyperglycaemia, inflammation or platelet aggregation.

European Patent Application No. 79301197.4, Publication No. 6735 (Beechams) discloses compounds of formula

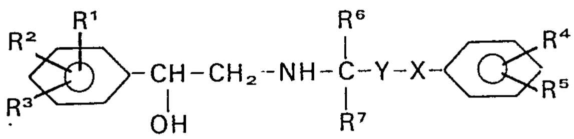

wherein

$R^1$ is hydrogen, fluorine, chlorine, hydroxyl, hydroxymethyl, methyl methoxy, amino, formamido, acetamido, methylsulphonamido, nitro, benzyloxy, methylsulphonylmethyl, ureido, trifluoromethyl or p-methoxybenzylamino;

$R^2$ is hydrogen, fluorine, chlorine or hydroxyl;

$R^3$ is hydrogen, fluorine, chlorine or hydroxyl;

$R^4$ is a carboxylic acid group or a salt, ester or amide thereof;

$R^5$ is hydrogen, fluorine, chlorine, methyl, methoxy, hydroxyl, or a carboxylic acid group or a salt ester or amide thereof;

$R^6$ is hydrogen, methyl, ethyl or propyl;

$R^7$ is hydrogen, methyl, ethyl or propyl;

X is oxygen or a bond; and

Y is $C_{1-16}$ alkylene or a bond,

which possess antiobesity and/or hypoglycaemic activity.

European Published Patent Application No. 21636 discloses compounds of Formula IA:

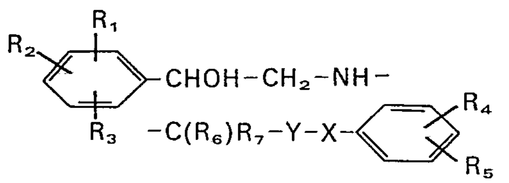

(IA)

or a pharmaceutically acceptable salt thereof, in which

$R_1$ is a hydrogen, fluorine, chlorine or bromine atom or a hydroxyl, hydroxymethyl, methyl, methoxyl, amino, formamido, acetamido, methylsulphonylamido, nitro, benzyloxy, methylsulphonylmethyl, ureido, trifluoromethyl or p-methoxybenzylamino group;

$R_2$ is a hydrogen, fluorine, chlorine or bromine atom or a hydroxyl group;

$R_3$ is a hydrogen, chlorine or bromine atom or a hydroxyl group;

$R_4$ is an alkyl group of 1 to 10 carbon atoms substituted by a hydroxyl, lower alkoxyl, oxo, lower acyloxy or $OCH_2CO_2H$ group or lower alkyl ester thereof;

$R_5$ is a hydrogen, chlorine or fluorine atom or a methyl, methoxyl or hydroxyl group or a carboxylic acid group or a salt, ester or amide thereof;

$R_6$ is a hydrogen atom or methyl, ethyl or propyl group;

$R_7$ is a hydrogen atom or a methyl, ethyl or propyl group;

X is an oxygen atom or a bond; and

Y is an alkylene groupe of up to 6 carbon atoms or a bond,

which also possess antiobesity and/or hypoglycaemic activity.

European Published Patent Application No. 7204 disloses compounds of Formula IB:

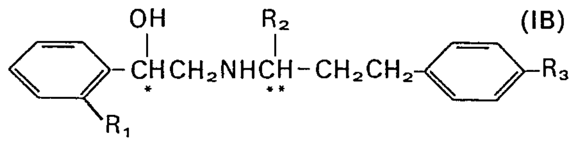

wherein

$R_1$ is hydrogen or fluorine;

$R_2$ is methyl or ethyl;

$R_3$ is hydroxy, $C_{1-4}$ alkanoyloxy, aminocarbonyl, methylaminocarbonyl or $C_{1-2}$ alkoxycarbonyl;

$\underset{*}{C}$ is an asymmetric carbon atom having the R absolute stereochemical configuration; and

$\underset{**}{C}$ is an asymmetric carbon atom having the S absolute stereochemical configuration;

or pharmaceutically acceptable salt thereof, which also possess antiobesity activity.

It has now been discovered that a class of novel aminoethanolamine derivatives have antiobesity and/or hypoglycaemic and/or anti-inflammatory and/or platelet aggregation inhibition activity. This activity is coupled with low cardiac stimulant activity for particular members of the class.

Accordingly, the present invention provides a compound of Formula I:

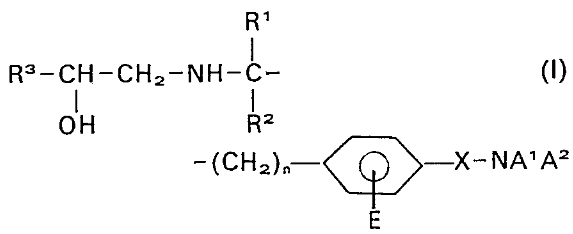

and salts thereof

wherein

$R^1$ is hydrogen or methyl;

$R^2$ is hydrogen or methyl;

$R^3$ is phenyl optionally substituted with one or two of the following: fluorine, chlorine, bromine, trifluoromethyl or $C_{1-6}$ alkyl, or is benzofuran-2-yl;

n is 1 or 2;

X is $C_{1-15}$ straight or branched alkylene;

$A^1$ is hydrogen, $C_{1-6}$ alkyl, or optionally substituted benzyl;

$A^2$ is hydrogen, $C_{1-6}$ alkyl, or optionally substituted benzyl; and

E is hydrogen, halogen, $C_{1-6}$ alkoxy, $C_{1-6}$ alkyl, or mono- or di-$C_{1-6}$ alkylamino.

A preferred sub-group of compounds within those or Formula I are those wherein

$R^3$ is phenyl optionally substituted by one of the following: fluorine, chlorine, bromine or trifluoromethyl;

X is $C_{1-6}$ straight or branched alkylene;

$A^1$ is hydrogen or $C_{1-6}$ alkyl;

$A^2$ is hydrogen or $C_{1-6}$ alkyl; and

E is hydrogen.

Preferably, n is 1.

Preferably, $R^1$ is hydrogen and $R^2$ is methyl.

Perferably one of $A^1$ or $A^2$ is hydrogen and the other is methyl.

Suitably X represents a methylene group.

Pharmaceutically acceptable salts of compounds of Formula I include acid addition salts formed with a pharmaceutically acceptable acid such as hydrochlorid acid, hydrobromic acid, orthophosphoric acid, sulphuric acid, methane sulphonic acid, toluenesulphonic acid, acetic acid, propionic acid, lactic acid, citric acid, fumaric acid, malic acid, succinic acid, salicylic acid or acetylsalicylic acid.

The salts of compounds of Formula I need not be pharmaceutically acceptable as they are also useful in the preparation of other compounds of Formula I and in the separation of stereo-isomers of compounds of Formula I when the salt ion is also optically active.

Compounds of Formula I have at least one asymmetric carbon atom, i.e. the one bearing the hydroxyl and $R^3$ groups, and when $R^1$ and $R^2$ are different, the carbon atom bearing $R^1$ and $R^2$ is also asymmetric. The compounds may therefore exist in at least two and often four stereo-isomeric forms. The present invention encompasses all stereo-isomers of the compounds of Formula I whether free from other stereo-isomers or admixed other stereo-isomers in any proportion and thus includes, for instance, racemic mixtures of enanetiomers.

Preferably, the carbon atom bearing the hydroxyl group and $R^3$ has the R configuration.

The most potent compounds of Formula I are those wherein $R^1$ and $R^2$ are different and both asymmetric carbon atoms are in the R configuration.

The absolute configuration of any compound of Formula I may be determined by conventional X-ray crystallographic techniques.

It is believed that, in the $^{13}C$ NMR of compounds of Formula I wherein one of $R^1$ and $R^2$ is hydrogen and the other is methyl, the diastereoisomer having the greater antiobesity activity is that for which the signal of the methyl group carbon atom appears at higher field (the lower numerical value when expressed in ppm) in $d_6$ DMSO solution. The paired resonances often appear at approcimately 20 ppm (less active) and slightly below 20 ppm (more active) down field from tetramethylsilane. Other paired resonances can occur for the carbon atoms attached directly to the ethanolamine nitrogen atom and the carbon which carries the hydroxyl group and $R^3$. Again the paired resonances of the more active diastereoisomer of the investigated compounds appear at the higher field position.

The present invention also provides a process for producing a compound of Formula I by reducing a compound of Formula II

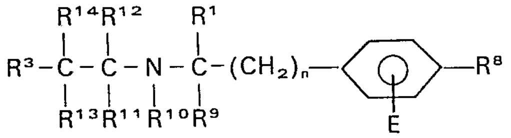 (II)

wherein

$R^1$, $R^3$, E and n are as defined in relation to Formula I;

$R^8$ is a group $-X-NA^1A^2$ or $-Y-Z$ wherein $A^1$, $A^2$ and X are as defined in relation to Formula I and Y is a bond or $C_{1-14}$ straight or branched alkylene, and Z is cyano or

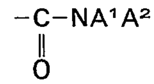

or

Y is $C_{1-15}$ straight or branched alkylene and Z is

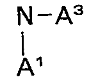

wherein $A^3$ is $C_{1-5}$ alkyl carbonyl.

$R^9$ is a group $R^2$ as defined in relation to Formula I or together with $R^{10}$ forms a bond;

$R^{10}$ is hydrogen, benzyl or together with $R^9$ or $R^{11}$ forms a bond;

$R^{11}$ is hydrogen or together with $R^{12}$ forms an oxo-group or together with $R^{10}$ forms a bond;

$R^{12}$ is hydrogen or together with $R^{11}$ forms an oxo-group;

$R^{13}$ is hydroxyl or together with $R^{14}$ forms an oxo-group;

$R^{14}$ is hydrogen or together with $R^{13}$ forms an oxo-group,

provided that there is no more than one oxo-group and no more than one bond represented by any of $R^9$ to $R^{14}$ and optionally thereafter forming a salt, of the compound of Formula I so formed and/or converting the compound of Formula I so formed into a further compound of Formula I.

When there are two or more reducible groups in the compound of Formula II, these may, generally, be reduced separately in any order or simultaneously.

Salts of compounds of Formula I may be produced directly using the appropriate compound of Formula II or may be produced from the free base of Formula I by conventional means. Free bases of Formula I may be obtained directly or from the corresponding salt by conventional means.

The compounds of Formula I so produced may be purified by conventional means such as crystallization and/or chromatography.

The aforementioned reductions may be effected by conventional chemical methods, such as reduction using a complex hydride, for instance lithium aluminium hydride, sodium cyanoborohydride or sodium borohydride, aluminium amalgam or boranemethyl sulphide or by catalytic hydrogenation using catalysts such as palladium on charcoal, or platinum, for instance, as platinum oxide.

Catalytic hydrogenation is conveniently effected using hydrogen gas at about 1 atm pressure when platinum is used as catalyst and at medium to high pressure, conveniently 50 to 100 psi, when

a palladium catalyst is used. Such hydrogenations may be conducted in conventional hydrogenation solvents such as a lower alkanol, for instance ethanol and at any convenient, non-extreme temperature. It is generally most suitable to use a slightly raised temperature such as 30 to 100° C, for example 40 to 80° C.

The desired compound may be isolated from the reaction mixture by evaporation of the filtered solution. The initially obtained product may be purified by conventional means, for example by chromatography, crystallization or the like.

Reduction by sodium borohydride is conveniently effected in a lower alkanolic solvent such as methanol or ethanol. The reaction is generally carried out at ambient or reduced temperatures of, for example, 0 to 30° C.

Reduction by lithium aluminium hydride is conveniently effected in a dry, ether solvent such as diethyl ether or tetrahydrofuran at ambient or elevated temperature.

The desired compound may be obtained, after reduction with either sodium borohydride or lithium aluminium hydride by hydrolysis of the reaction mixture, extraction into a suitable solvent such as ethyl acetate and evaporation. The initially obtained product may be purified as outlined hereinbefore.

In particular aspects, the present invention provides processes for producing compounds of Formula I by reducing a compound of Formula IIA

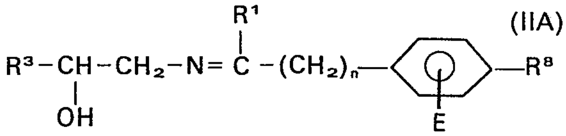
(IIA)

or reducing a compound of Formula IIB

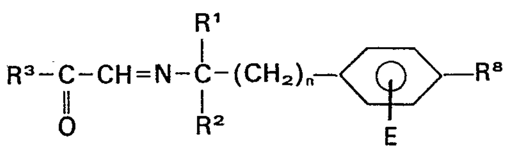
(IIB)

or reducing a compound of Formula IIC

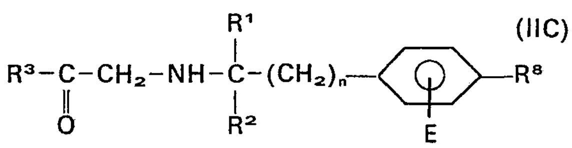
(IIC)

or the N-benzyl derivative thereof, or reducing a compound of Formula IID

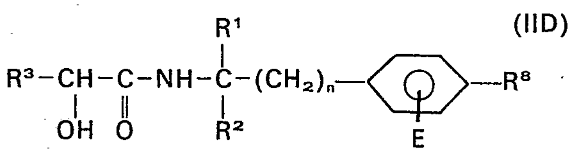
(IID)

or reducing a compound of Formula IIE

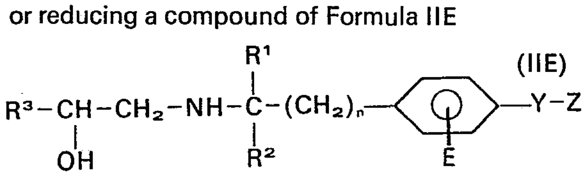
(IIE)

wherein $R^1$, $R^2$, $R^3$, $A^1$, $A^2$ and $R^8$, Y, E, Z, and n are as defined in relation to Formulae I and II.

The present invention also provides a process for producing a compound of Formula I by reacting a compound of Formula III

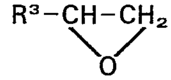
(III)

with a compound of Formula IV

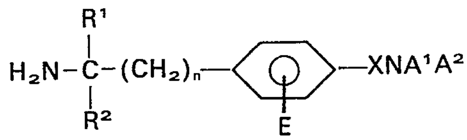
(IV)

wherein $R^1$, $R^2$, $R^3$, E, $A^1$, $A^2$, X and n are as defined in relation to Formula I; and optionally thereafter forming a salt, of the compound of Formula I so formed and/or converting the compound of Formula I so formed into a further compound of Formula I.

This reaction is conveniently effected in a solvent such as a lower alkanol, preferably ethanol.

Compounds of formula II may be produced by reacting a compound of Formula V

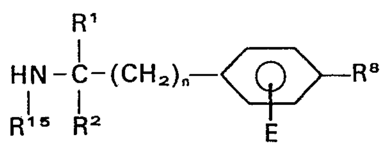
(V)

wherein $R^1$, $R^2$, E, $R^8$ and n are as defined in relation to Formulae I and II and $R^{15}$ is hydrogen or benzyl with a compound of Formula VI

$$R^3-W \qquad (VI)$$

wherein W contains a group which is capable of reacting with the amine of Formula V thus forming a compound of Formula II. Typical examples of compounds of Formula VI are

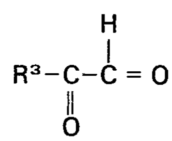
(VIA)

or its hydrate or hemiacetal of a lower alkanol;

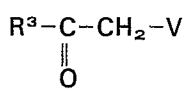
(VIB)

wherein V is a halogen atom, preferably bromine;

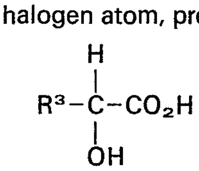
(VIC)

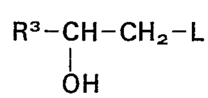
(VID)

wherein L is a leaving group, such as halogen or preferably tosyloxy;

and 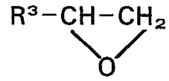 (III)

The compounds of Formulae VIA and VIC are only suitable for reacting with a compound of Formula V when $R^{15}$ in Formula V is hydrogen.

It will be appreciated that when $R^8$ is a group $-XNA^1A^2$ and $R^{15}$ is hydrogen, the reaction of a compound of Formula V with the compound of Formula VID or III produces a compound of Formula I directly, this being identical to the reaction between compounds of Formulae III and IV above.

Conventional conditions suitable for use with the particular compound of Formula VI may be used for this reaction. Thus the reaction of a compound of Formula VIA with a compound of Formula V is conveniently conducted at elevated temperature under conditions resulting in the removal of the water formed during the reaction. A particularly suitable method is to perform the reaction in a solvent such as benzene, under reflux and azeotropically to remove the water using a Dean and Stark trap.

The reaction of a compound of Formula VIB with a compound of Formula V is conveniently conducted in a polar organic solvent such as acetonitrile or butanone, at an elevated temperature, for instance under reflux.

The reaction of a compound of Formula VIC with a compound of Formula V is conveniently conducted under standard peptide formation reaction conditions.

The reaction of a compound of Formula VID with a compound of Formula V is conveniently conducted in a solvent such as dimethyl sulphoxide at elevated temperature, preferably 50° C for about 3 d.

The reaction of a compound of Formula III with a compound of Formula V is conveniently conducted in a solvent such as a lower alkanol, preferably ethanol.

A particularly preferred process for the production of compounds of Formula II comprises reacting a compound of Formula VII

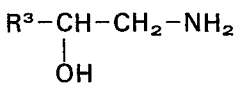 (VII)

with a compound of Formula VIII

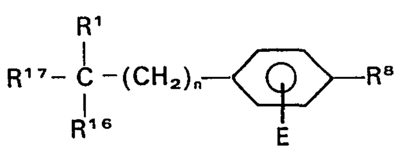 (VIII)

wherein

$R^1$, $R^8$, E and n are as defined in relation to Formula I or II

$R^{16}$ is hydrogen or together with $R^{17}$ is oxo, and

$R^{17}$ is a leaving group, such as halogen, or preferably tosyloxy, or together with $R^{16}$ is oxo.

The reaction of a compound of Formula VII with a ketone of Formula VIII is conveniently effected under conditions which result in the removal of water formed during reaction. A particularly suitable method is to perform the reaction in a solvent, such as benzene, under reflux and azeotropically to remove the water using a Dean and Stark trap.

The reaction of a compound of Formula VII with a compound of Formula VIII wherein $R^{17}$ is a group L is conveniently effected in a solvent such as dimethylsulphoxide at elevated temperature, preferably at about 50° C for about two to three days.

It is often convenient to prepare the compound of Formula II and then use it *in situ* without isolation to produce the required compound of Formula I.

The intermediates of Formulae III, IV, V, VI and VII may be produced by conventional methods.

Those compounds of Formula I having only one asymmetric carbon atom (i.e. when $R^1$ and $R^2$ are the same) may, if desired, be resolved into enantiomers by conventional means, for example by the use of an optically active acid as a resolving agent. Those compounds of Formula I having two asymmetric carbon atoms may be separated into diastereomeric pairs of enantiomers by, for example, fractional crystallization from a suitable solvent such as ethyl acetate or benzene. The pair of enantiomers thus obtained may be separated into individual stereo-isomers by conventional means such as by the use of an optically active acid as a resolving agent.

Suitable optically active acids which may be used as resolving agents are described in "Topics in Stereochemistry", vol. 6, Wiley Interscience, 1971; Allinger N.L. and Eliel W.L. (Eds.).

Any desired enantiomer of a compound of Formula I may be obtained by stereospecific synthesis using optically pure starting materials of known configuration. Thus, by using single enantiomers of the compounds of Formulae III and IV a required enantiomer of the corresponding compound of Formula I can be obtained. Similarly, the reaction of a single enantiomer of a compound of Formula V with a single enantiomer of Formula VIC below will result in a single enantiomer of a compound of Formula IID above. The latter may be reduced to a compound of Formula I without alterning the stereochemical configuration thereby affording a single enantiomer of the compound of Formula I. Reaction of a compound of Formula V with a single enantiomer of a compound of Formula VID will give a single enantiomer of a compound of formula I directly. Thus, for instance, the R enantiomer of a compound of Formula VID or VIC, when reacted with the R enantiomer of a compound of Formula V directly or indirectly affords the RR enantiomer of the desired compound of Formula I.

Reduction of a single optical isomer of a compound of Formula IIE, wherein $-Y-Z$ is the only reducible moiety, results in the production of the corresponding single optical isomer of a compound of Formula I. Thus, for example, reduction of the R,R-stereo-isomer of a compound of Formula IIE wherein Y is a bond and Z is

$-CNHCH_3$
‖
O

affords the R,R-optical isomer of a compound of Formula I wherein $X-NA^1A^2$ is $CH_2NHCH_3$.

Many of the reductions described in relation to compound of Formula II directly involve the asymmetric, or potentially asymmetric carbon atoms and consequently result in the formation of mixtures of stereo-isomers. Thus reduction of a single enantiomer of a compound of Formulae IIA, IIB or IIC will result in the formation of a pair of enantiomers of a corresponding compound of Formula I whereas reduction of a racemic mixture of enantiomers of a compound of Formulae IIA, IIB or IIC will result in the formation of a mixture of all four enantiomers of the corresponding compound of Formula I. Such mixtures may be separated into pairs of enantiomers and/or resolved into single enantiomers as described above.

A compound of Formula I, or pharmaceutically acceptable salt thereof, where appropriate, (hereinafter the drug) may be administered as the pure drug; however, it is preferred that the drug be administered as a pharmaceutical composition also comprising a pharmaceutically acceptable carrier.

Accordingly, the present invention also provides a pharmaceutical composition comprising a compound of Formula I or pharmaceutically acceptable salt, ester or amide thereof, where appropriate, in association with a pharmaceutically acceptable carrier therefore.

As used herein the terms pharmaceutical composition and pharmaceutically acceptable embrace compositions and ingredients for both human and/or veterinary use.

Usually the compositions of the present invention will be adapted for oral administration although compositions for administration by other routes, such as topically or by injection are also envisaged.

Particularly suitable compositions for oral adminitration are unit dosage forms such as tablets and capsules. Other fixed-unit dosage forms, such as powders presented in sachets, may also be used.

In accordance with conventional pharmaceutical practice the carrier may comprise a diluent, binder, filler, disintegrant, wetting agent, lubricant, colourant, flavourant of the like.

Typical carriers may, therefore, comprise such agents as microcrystalline cellulose, starch, sodium starch glycollate, polyvinylpyrrolidone, polyvinylpolypyrrolidone, magnesium stearate, sodium lauryl sulphate, sucrose and the like.

Most suitably the composition will be provided in unit dose form. Such unit doses will normally comprise 0.1 to 500 mg, more usually 0.1 to 150 mg and favourably 0.1 to 100 mg. Such doses may be taken one to six times a day in a manner such that the total daily dose for a 70-kg adult will generally be about 0.1 to 1000 mg and more usually about 1 to 500 mg.

In addition to use in human medicine the compositions of this invention may be used to treat obesity, hyperglycaemia, inflammation and to inhibit platelet aggregation in mammals, especially domestic mammals, such as dogs. In general, administration to domestic mammals may be by mouth and will usually take place one or two times a day at about 0.025 mg/kg to 10 mg/kg, for example 0.1 mg/kg to 2 mg/kg.

The invention will now be illustrated by references to the following examples, which are not intended to limit the invention in any way. The preparation of intermediates is described in the descriptions. All melting points are given in degrees centigrades.

*Example 1:*

*N-[2-(4-Aminomethylphenyl)-1-methylethyl]-2-hydroxy-2-phenylethanamine*

N-[2-(4-Cyanophenyl)-1-methylethyl]-2-hydroxy-2-phenylethanamine (3.33 g of a 77:23 mixture) in dry tetrahydrofuran was added dropwise under nitrogen to a suspension of lithium aluminium hydride (0.8 g) in dry tetrahydrofuran. The mixture was refluxed for 1 h at the end of addition. Water (0.8 ml), 2N sodium hydroxide (0.8 ml) and further water (2.4 ml) were added, the mixture filtered and the filtrate evaporated to give an oil (3.8 g). This was dissolved in ether and treated with ethereal hydrogen chloride to give the title compound as the dihydrochloride salt, isolated as an 80:20 mixture of diastereo-isomers, m.p. 251-260 (methanol/ethyl acetate).

$\tau$ ($d_6$ DMSO): 8.85 (3H, d, J=6Hz), 6.2-7.4 (5H, m), 6.05 (2H, s), 4.85 (1H, m), 3.7 (1H, broad, disappears with $D_2O$), 2.3-2.8 (9H, m), (1.3 (2H) + 0.9 (2H) + 0.1 (1H), all disappear with $D_2O$).

*Example 2:*

*N-[2-(4-Aminomethylphenyl)-1-methylethyl]-2-hydroxy-2-(3-trifluoromethylphenyl)ethanamine*

This was prepared in an identical manner to the compound described in Example 1 using N-[2-(4-cyanophenyl)-1-methylethyl]-2-hydroxy-2-(3-trifluoromethylphenyl)ethanamine. The title compound was isolated as the dihydrochloride salt as a 50:50 mixture of diastereo-isomers, m.p. 227-232 (methanol/ethyl acetate).

$\tau$ ($d_6$ DMSO): 8.8 (3H, d, J=6Hz), 6.3-7.3 (5H, m), 6.0 (2H, s), 4.7 (1H, m), 3.5 (1H, broad, disappears with $D_2O$), 2.65 (2H, d, J=9Hz), 2.45 (2H, d, J=9Hz), 2.1-2.4 (4H, m), (1.25 (2H) + 0.8 (2H) + 0.0 (1H), all disappears with $D_2O$).

*Example 3:*

*N-[2-(4-N'-Methylaminomethylphenyl)-1-methylethyl]-2-hydroxy-2-(3-trifluoromethyl-phenyl)ethanamine*

N-[2-(4-N'-Methylcarboxamidophenyl)-1-methylethyl]-2-hydroxy-2-(3-trifluoromethylphenyl)ethanamine (0.89 g of a 53:47 mixture) in dry tetrahydrofuran was added dropwise to lithium aluminium hydride (0.2 g) in dry tetrahydrofuran. The mixture was refluxed for 1 H at the end of addition. Water (0.2 ml), 2N sodium hydroxide

(0.2 ml) and further water (0.6 ml) were added and the mixture filtered. The filtrate was evaporated and the title compound isolated as a 59:41 mixture of diastereo-isomers as the dihydrochloride salt, m.p. 258-264 (methanol/ethyl acetate).

τ ($d_6$ DMSO): 8.85 (3H, d, J=6Hz), 6.2-7.3 (8H, m), 5.9 (2H, s), 4.75 (1H, m), 3.5 (1H, disappears with $D_2O$), 2.7 (2H, d, J=9Hz), 2.45 (2H, d, J=9Hz), 2.15-2.4 (4H, m), (0.9 (1H) + 0.4 (2H) + 0.1 (1H), all disappear with $D_2O$).

*Example 4:*

*N-[2-(4-N'N'-Dimethylaminomethylphenyl)-1-methylethyl]-2-hydroxy-2-(3-trifluoromethylphenyl)ethanamine*

This was prepared in an identical manner to the compound described in Example 3 using N-[2-(4-N'N'-dimethylcarboxamidophenyl)-1-methylethyl]-2-hydroxy-2-(3-trifluoromethylphenyl)-ethanamine. The title compound was isolated as dihydrochloride salt as a 48:52 mixture of diastereo-isomers, m.p. 213-225 (methanol/ethyl acetate).

τ ($d_6$ DMSO): 8.8 (3H, d, J=6Hz), 7.25 (6H, s), 6.2-7.3 (5H, m), 5.75 (2H, s), 4.7 (1H, m), 3.5 (1H, disappears with $D_2O$), 2.65 (2H, d, J=9Hz), 2.3 (2H, d, J=9Hz), 2.0-2.3 (4H, m), 1.0-1.0 (3H, m).

*Example 5:*

*N-[2-(4-Methylaminomethylphenyl)-1-methylethyl]-2-hydroxy-2-(2-benzofuranyl)ethanamine*

N-[2-(4-N'-Methylcarboxamidophenyl)-1-methylethyl]-2-hydroxy-2-(2-benzofuranyl)-ethanamine (5.4 g) in dry tetrahydrofuran was added to lithium aluminium hydride (0.6 g) in dry tetrahydrofuran. The mixture was heated under reflux for 1 h. Water (0.6 ml), 2N sodium hydroxide (0.6 ml) followed by further water (1.8 ml) was added, the mixture filtered and the filtrate evaporated to give an oil. N-[2-(4-Methylaminomethylphenyl)-1-methylethyl]-2-hydroxy-2-(2-benzofuranyl)ethanamine was isolated as the dihydrochloride salt, m.p. 129-263 (ethylacetate/methanol) as a 27:73 mixture of diastereo-isomers.

τ ($d_6$ DMSO): 8.85 (3H, d, J=6Hz), 7.1-7.4 (3H, m), 6.2-6.9 (5H, m, + 1H, disappears with $D_2$), 5.9 (2H, s), 4.7 (1H, m), 3.1 (1H, s), 2.6-2.9 (4H, m), 2.25-2.55 (4H, m), (0.6-1.0 (1H), 0.4 (2H) + 0.0 (1H), all disappear with $D_2O$).

*Example 6:*

*N-[2-(4-Aminomethylphenyl)-1-methylethyl]-2-hydroxy-2-(3-chlorophenyl)ethanamine*

This was prepared in an identical manner to the compound described in Example 1 using N-[2-(4-cyanophenyl)-2-methylethyl]-2-hydroxy-2-(3-chlorophenyl)ethanamine. The title compound was islolated as the dihydrochloride salt as a 49:51 mixture of disastereo-isomers, m.p. 230-238 (methanol/ethyl acetate).

τ ($d_6$ DMSO): 8.8 (3H, d, J=6Hz), 6.2-7.3 (5H, m), 6.0 (2H, s), 4.8 (1H, m), 4.0-3.2 (1H, broad, disappears with $D_2O$), 2.3-2.75 (8H, m), 1.2 (3H, s, disappears with $D_2O$), 0.85 (1H, broad, disappears with $D_2O$), 0.1 (1H, broad, disappears with $D_2O$).

*Example 7:*

*N-[2-(4-N'-Methylaminomethylphenyl)-1-methylethyl]-2-hydroxy-2-phenylethanamine*

This was prepared in an identical manner to the compound described in Example 3 using N-[2-(4-N'-methylcarboxymidophenyl)-1-methylethyl]-2-hydroxy-2-phenylethanamide. The title compound was isolated as the dihydrochloride salt as a 55:45 mixture of diastereo-isomers, m.p. 235-246 (methanol/ethyl acetate).

τ ($d_6$ DMSO): 8.85 (3H, d, J=6Hz), 7.5 (3H, s), 6.3-7.3 (5H, m), 5.9 (2H, s), 4.8 (1H, m), 3.75 (1H, s, disappears with $D_2O$), 2.3-2.9 (8H, m), 0.35 (4H, broad s, disappears with $D_2O$).

*Example 8:*

*N-[2-(4-N'-Methylaminomethylphenyl)-1-methylethyl]-2-hydroxy-2-(3-chlorophenyl)-ethanamine*

This was prepared in an identical manner to the compound described in Example 3 using N-[-2-(4-N'-methylcarboxamidophenyl)-1-methylethyl]-2-hydroxy-2-(3-chlorophenyl)ethanamine. The title compound was isolated as the dihydrochloride salt as a 67:33 mixture of diastereo-isomers, m.p. 269-278 (methanol/ethyl acetate).

τ ($d_6$ DMSO): 8.8 (3H, d, J=6Hz), 7.5 (3H, s), 6.1-7.3 (5H, m), 5.9 (2H, s), 4.8 (1H, m), 3.6 (1H, broad s, disappears with $D_2O$), 2.3-2.8 (8H, m), 0.9 (1H, broad, disappears with $D_2O$), 0.0-0.65 (3H, broad, disappears with $D_2O$).

*Example 9:*

*N-[2-(4-N'-Methylaminomethylphenyl)-1-methylethyl]-2-hydroxy-2-(3-fluorophenyl)-ethanamine*

A mixture of 2-hydroxy-2-(3-fluorophenyl)-ethanamine (1.53 g) and (4-N-methylaminomethylphenyl)-propan-2-one (1.75 g) was heated under reflux in benzene using a Dean and Stark apparatus until the theoretical amount of water had been evolved. The solvent was removed, replaced with methanol and sodium borohydride (1.5 g) added portionwise. The methanol was evaporated, the residue partioned between ether and water and the combined ether layers dried, evaporated and the residue chromatographed on Kieselgel 60. Elution with chloroform/methanol/ammonia (92:7:1) gave an oil which was taken up in ethyl acetate, ethereal hydrogen chloride added and the title compound isolated as the dihydrochloride salt as a 61:39 mixture of diastereoisomers, m.p. 250-260 (methanol/ethyl acetate).

τ ($d_6$ DMSO): 8.8 (3H, d, J=6Hz), 7.5 (3H, s), 6.2-7.35 (5H, s), 5.95 (2H, s), 4.6 (1H, m), 3.6 (1H, broad, disappears with $D_2O$), 2.1-2.9 (8H, m), 0.9 (1H, broad, disappears with $D_2O$), 0.0-0.7 (3H, m, disappears with $D_2O$).

*Example 10:*

*N-[2-(4-N'-Methylaminomethylphenyl)-1-methylethyl-2-hydroxy-2-(3-ethylphenyl)-ethanamine*

This was prepared in an identical manner to the compound described in Example 9 using 2-hydroxy-2-(3-ethylphenyl)ethanamine and (4-N-methylaminomethylphenyl)propan-2-one. The title compound was isolated as the dihydrochloride salt as a 56:44 mixture of diastereoisomers, m.p. 218-238 (methanol/ethyl acetate).

τ ($d_6$ DMSO): 8.8 (3H, d, J=6Hz), 8.75 (3H, t, J=7Hz), 7.45 (3H, s), 7.4 (2H, q, J=7Hz), 6.2-7.2 (5H, m), 5.9 (2H, s), 4.9 (1H, m), 3.8 (1H, s, disappears with $D_2O$), 2.5-3.0 (6H, m), 2.45 (2H, d, J=7Hz), 0.35 (4H, broad, disappears with $D_2O$).

*Example 11:*

*N-[2-(4-N'-Methylaminomethylphenyl)-1-methylethyl]-2-hydroxy-2-(2-fluorophenyl)-ethanamine*

This was prepared in an identical manner to the compound described in Example 9 using 2-hydroxy-2-(2-fluorophenyl)ethanamine and (4-N-methylaminomethylphenyl)propan-2-one. The title compound was isolated as the dihydrochloride salt as a 50:50 mixture of diastereoisomers, m.p. 252-258 (methanol/ethyl acetate).

τ ($d_6$ DMSO): 8.85 (3H, d, J=6Hz), 7.5 (3H, s), 6.35-7.4 (5H, m), 5.95 (2H, s), 4.85 (1H, m), 3.65 (1H, disappears with $D_2O$), 2.6-3.0 (6H, m), 2.45 (2H, d, J=7Hz), 0.5 (4H, disappears with $D_2O$).

*Example 12:*

*N-[2-(4-N'-Methylaminomethylphenyl)-1-methylethyl]-2-hydroxy-2-(3-bromophenyl)-ethanamine*

This was prepared in an identical manner to the compound described in Example 9 using 2-hydroxy-2-(3-bromophenyl)ethanamine and (4-N-methylaminomethylphenyl)propan-2-one. The title compound was isolated as the dihydrochloride salt as a 51:49 mixture of diastereoisomers m.p. 234-263 (methanol/ethyl acetate).

τ ($d_6$ DMSO): 8.8 (3H, d, J=6Hz), 7.5 (3H, s), 6.2-7.5 (5H, m), 5.95 (2H, s), 4.9 (1H, m), 3.6 (1H, broad, disappears with $D_2O$), 2.2-2.8 (8Hz, m), 0.95 (1H, broad, disappears with $D_2O$), 0.4 (3H, broad, disappears with $D_2O$).

*Example 13:*

*N-[2-(4-N'-Ethylaminomethylphenyl)-1-methylethyl]-2-hydroxy-2-(3-trifluoromethylphenyl)-ethanamine*

A solution of N-[2-(4-N'-acetylaminomethylphenyl)-1-methylethyl]-2-hydroxy-2-(3-trifluoromethylphenyl)ethanamine (0.75 g) in dry tetrahydrofuran was added to lithium aluminium hydride (0.2 g) in dry tetrahydrofuran under nitrogen. The solution was heated at reflux for 1H after the end of addition and then cooled in ice. Water (0.2 ml), 2N sodium hydroxide (0.2 ml) followed by more water (0.6 ml) was then added, the solution filtered, dried and evaporated to give an oil from which the title compound was isolated as the dihydrochloride salt as a 39:61 mixture of diastereo-isomers, m.p. 210-226.

τ ($d_6$ DMSO): 8.8 (3H, d, J=6Hz), 8.75 (3H, t, J=7Hz), 7.05 (2H, q, J=7Hz), 6.2-6.9 (5H, m), 5.9 (2H, s), 4.7 (1H, m), 3.5 (1H, broad, disappears with $D_2O$), 2.1-2.7 (8H, m), 0.85 (1H, broad, disappears with $D_2O$), 0.4 (2H, broad, disappears with $D_2O$), 0.1 (1H, broad, disappears with $D_2O$).

*Example 14:*

*N-{2-[4-(3-N'-Methylaminopropyl)phenyl]-1-methylethyl}-2-hydroxy-2-(3-trifluoromethyl-phenyl)ethanamine*

This was prepared in an identical manner to the compound described in Example 3 using N-{2-[4-(2-N'-methylcarboxamido-ethyl)phenyl]-1-methylethyl}-2-hydroxy-2-(3-trifluoromethyl-phenyl)ethanamine. The title compound was isolated as the dihydrochloride salt as a 90:10 mixture of diastereo-isomers, m.p. 238-242 (methanol/ethyl acetate).

τ ($d_6$ DMSO): 8.8 (3H, d, J=6Hz), 7.9-8.4 (2H, m), 7.5 (3H, s), 7.0-7.5 (5H, m), 6.2-7.0 (4H, m), 4.75 (1H, m), 3.55 (1H, broad, disappears with $D_2O$), 2.75 (4H, s), 2.1-2.5 (4H, m), 0.8 (3H, broad, disappears with $D_2O$), 0.2 (1H, broad, disappears with $D_2O$).

*Example 15:*

*(RR,SS) N-[2-(4-N'-Methylaminomethylphenyl)-1-methylethyl]-2-hydroxy-2-(3-trifluoromethylphenyl)ethanamine*

The compound of Example 3 was recrystallized twice from methanolethylacetate to give a sample of 87% (RR,SS): 13% (RS,SR) ratio of diastereoisomers. This sample was recrystallized twice from ethanol to give a sample, m.p. 269-272 with a 93% (RR,SS): 7% (RS,SR) ratio of diastereo-isomers.

*Example 16:*

*(RR,SS) N-[2-(4-N'-Methylaminomethylphenyl-1-methylethyl]-2-hydroxy-2-(3-chlorophenyl)ethanamine*

A mixture of 2-hydroxy-2-(3-chlorophenyl-ethanamine (30.3 g) and (4-carbomethoxyphenyl)propan-2-one (34.0 g) was heated under reflux in benzene using a Dean and Stark apparatus until the theoretical amount of water had been evolved. The solvent was removed, replaced with methanol and sodium borohydride (30 g) added portionwise. The methanol was evaporated, the residue partitioned between ether and water and the combined ether layers dried. The solution was filtered, treated with ethereal hydrogen bromide and the ether decanted from the precipitated gum. The gum was taken up in hot ethyl acetate and the solution allowed to cool. The deposited crystals were filtered to give N-[2-(4-carbomethoxyphenyl)-1-methylethyl]-2-hydroxy-2(3-chlorophenyl)ethanamine hydrobromide (23 g) as an 86:14 ratio of diastereo-isomers. Recrystallization of this sample from methanol/ethyl acetate gave 16 g as a 99:1 ratio of (RR,SS):(RS,SR) diastereo-isomers.

This sample was converted to the free base with 2N sodium hydroxide solution and dissolved in ethanolic methylamine (approx. 15 ml) and heated at 100°C for 5 h in an autoclave. The crude amide was isolated by evaporation of the solvent, not purified further but dissolved in dry tetrahydrofuran and added dropwise to lithium aluminium hydride (4.5 g) in tetrahydrofuran. The solution was refluxed for 1 h at the end of addition. Water (4.5 ml), 2N sodium hydroxide (4.5 ml) followed by more water (13.5 ml) were added and the mixture filtered. The filtrate was evaporated and the residual oil chromatographed on Kieselgel 60. Elution with methanol chloroform: ammonia (10:89:1) gave an oil (11.5 g). Conversion of this to the dihydrochloride salt and recrystallization from methanol/ethyl acetate gave the tilte compound dihydrochloride, 6.0 g, as a 98:2 mixture of diastereo-isomers, m.p. 284-286.$^1$HNMR identical with that of Example 8.

*Example 17:*

*(RR) N-[2-(4-N'-Methylaminomethylphenyl)-1-methylethyl]-2-hydroxy-2-(3-chlorophenyl)-ethanamine*

This enantiomer was prepared from N-[2-(4-N'-methylcarboxamidophenyl)-1-methylethyl]-2-hydroxy-2-(3-chlorophenyl)ethanamine (98% RR) in an identical manner to the procedure described for the preparation of the diastereoisomer in Example 16. The title compound was isolated as the dihydrochloride salt m.p. 296-298 (methanol/ethyl acetate), $[\alpha]_D^{25}$ $H_2O$-78.47.

*Example 18:*

*N-[3-(4-N'-Methylaminomethylphenyl)-1,1-dimethylpropyl]-2-hydroxy-2-(3-chlorophenyl)-ethanamine*

This was prepared in an identical manner to the compound described in Example 3 using N-[-(4-N'-methylcarboxamidophenyl)-1,1-dimethylpropyl]-2-hydroxy-2-(3-chlorophenyl)ethanamine. The title compound was isolated as the dihydrochloride salt m.p. 201-204 (methanol/ethyl acetate).

$\tau$ ($d_6$ DMSO): 8.6 (6H, s), 7.8-8.3 (2H, m), 7.52 (3H, s), 7.1-7.6 (2H, m), 6.9 (2H, m), 5.95 (2H, s), 4.9 (1H, m), 3.6 (1H, m, disappears with $D_2O$), 2.3-2.8 (8H, m), 1.0-1.5 (1H, broad, disappears with $D_2O$), 0.45 (2H, s, disappears with $D_2O$), 0.0-0.6 (1H, broad, disappears with $D_2O$).

*Example 19:*

*N-[2-(4-N'-Methylaminomethylphenyl)ethyl]-2-hydroxy-2-(3-trifluoromethylphenyl)ethanamine*

This was prepared in an identical manner to the compound described in Example 16 using N-[2-(4-carbomethoxyphenyl)ethyl]-2-hydroxy-2-(3-trifluoromethylphenyl)ethanamine. The title compound was isolated as the dihydrochloride salt m.p. 268-273 (methanol/ethyl acetate).

$\tau$ ($d_6$ DMSO): 7.5 (3H, s), 6.4-7.3 (6H, m), 5.9 (2H, s), 4.75 (1H, m), 3.52 (1H, s, disappears with $D_2O$), 2.1-2.8 (8H, m), 0.3 (4H, broad s, disappears with $D_2O$).

*Example 20:*

*(RR,SS) N-[2-(4-N'-Benzylaminomethylphenyl)-1-methylethyl]-2-hydroxy-2-phenylethanamine*

N-[2-(4-Carbomethoxyphenyl)-1-methylethyl]-2-hydroxy-2-phenylethanamine (2.0 g) was heated under reflux in benzyl amine (15 ml) until tlc showed no starting ester (approx. 30 h). Evaporation of the solvent and trituration of the residue with hexane gave the amide as a solid (2.54 g) which was not purified further but dissolved in tetrahydrofuran and reduced with lithium aluminium hydride according to the procedure of Example 3. Chromatography on Kieselgel 60 ($MeOH/CHCl_3/NH_3$; 7:92:1) gave an oil from which the title compound was isolated as a 97:3 mixture of diastereo-isomers as the dihydrochloride salt, 0.8 g, m.p. 238-247 (methanol/ethyl acetate).

$\tau$ ($d_6$ DMSO): 8.85 (3H, d, J=6Hz), 6.15-7.4 (5H, m), 5.9 (4H, s), 4.85 (1H, m), 3.75 (1H, s, disappears with $D_2O$), 2.2-2.8 (14H, m), 0.85 (1H, broad, disappears with $D_2O$), 0.0 (3H, broad, disappears with $D_2O$).

*Example 21:*

*N-[2-(4-N'-Methylaminomethylphenyl)-1-methylethyl]-2-hydroxy-2-(3-chloro-2-fluorophenyl)ethanamine*

This was prepared in an identical manner to the compound described in Example 9 using 2-hydroxy-2-(3-chloro-2-fluorophenyl)ethanamine and (4-N-methylaminomethylphenyl)propan-2-one. The title compound was isolated as the dihydrochloride salt as a 49:51 mixture of diastereo-isomers, m.p. 259-261 (methanol/ethyl acetate).

$\tau$ ($d_6$ DMSO): 8.8 (3H, d, J=6Hz), 7.55 (3H, s), 6.2-7.4 (5H, m), 5.9 (2H, s), 4.55 (1H, m), 3.45 (1H, disappears with $D_2O$), 2.25-2.8 (7H, m), 0.9 (1H, disappears with $D_2O$), 0.4 (2H, disappears with $D_2O$), 0.1 (1H, disappears with $D_2O$).

*Example 22:*

*N-{2-[4-(2-N'-Methylaminoethyl)phenyl]-1-methylethyl}-2-hydroxy-2-(3-chlorophenyl)-ethanamine*

A mixture of 2-hydroxy-2-(3-chlorophenyl)-ethanamine (1.45 g) and 4-[2-(N-formylaminoethyl)]phenylpropan-2-one (1.74 g) was heated under reflux in benzene using a Dean and Stark apparatus until the theoretical amount of water had been evolved. The benzene was evaporated, replaced with methanol and sodium borohydride (1 g) added portionwise. The solvent was removed, the residue partitioned between water and ether and the ether layers combined and dried.

Evaporation gave the N-formyl compound (3.10 g) which was not purified further but dissolved in dry tetrahydrofuran and added dropwise to lithium aluminium hydride (0.34 g) in dry tetrahydrofuran. The solution was refluxed for 1 h at the end of addition. Water (0.3 ml), 2N sodium hydroxide (0.3 ml) followed by more water (0.9 ml) was added, the mixture filtered and the filtrate evaporated to give an oil which was chromatographed on Kieselgel 60. Elution with methanol/ammonia/chloroform (9:1:90) gave the title compound as an oil which was isolated as the dihydrobromide salt as a 51:49 mixture of diastereo-isomers, m.p. 204-208 (methanol/ethyl acetate).

τ ($d_6$ DMSO): 8.85 (3H, d, J=6Hz), 7.4 (3H, s), 6.2-7.3 (9H, m), 4.9 (1H, m), 3.7 (1H, disappears with $D_2O$), 2.7 (4H, s), 2.4-2.6 (4H, m), 1.2 (4H, broad, disappears with $D_2O$).

*Example 23:*

*N-[2-{4-[1-(S)-N'-Methylaminoethyl]phenyl}-1-methylethyl]-2-hydroxy-2-(3-chlorophenyl)-ethanamine*

This was prepared in an identical manner to the compound described in Example 22 replacing 4-[2-(N-formylaminoethyl)phenyl]propan-2-one by 4-[1-(S)-N-formylaminoethyl]phenylpropan-2-one. The title compound was isolated as the dihydrobromide salt as a 48:52 mixture of diastereo-isomers, m.p. 237-242 (methanol/ethyl acetate) $[\alpha]^{25}_{D} H_2O$ −9.2.

τ ($d_6$ DMSO): 8.8 (3H, d, J=6Hz), 8.45 (3H, d, J=6Hz), 7.6 (3H, s), 6.1-7.4 (5H, m), 5.6 (1H, broad, sharpens to quartet, J=6Hz, $o_n$ $D_2O$), 4.85, (1H, m), 3.7 (1H, disappears with $D_2O$), 2.3-2.7 (8H, m), 0.9 (4H, broad, disappears with $D_2O$).

*Example 24:*

*N-[2-{4-[1-(R)-N'-Methylaminoethyl]phenyl}-1-methylethyl]-2-hydroxy-2-(3-chlorophenyl)-ethanamine*

This was prepared in an identical manner to the compound described in Example 23 replacing 4-[1-(S)-N-formylaminoethyl]phenylpropan-2-one by its (R)-isomer. The title compound was isolated as the dihydrobromide salt as a 47:53 mixture of diastereo-isomers, m.p. 204-227 (methanol/ethyl acetate), $[\alpha]^{25}_{D} H_2O$ +10.45.

τ ($d_6$ DMSO): identical with the (S) isomer of Example 23, apart from slight variation in position of the replaceable protons.

*Example 25:*

*N-{2-[4-(2-N'-Methylaminopropyl)phenyl]-1-methylethyl}-2-hydroxy-2-(3-chlorophenyl)-ethanamine*

This was prepared in an identical manner to the compound described in Example 22 replacing 4-[2-(N-formylamino)ethyl]phenylpropan-2-one by 4-(2-N-formylaminopropyl)phenylpropan-2-one. The title compound was isolated as the dihydrobromide salt as a 52:48 mixture of diastereo-isomers, m.p. 202-207 (methanol/ethyl acetate).

τ ($d_6$ DMSO): 8.95 (6H, m), 7.2-7.5 (6H, m), 6.2-7.0 (5H, m), 5.15 (1H, broad, disappears with $D_2O$), 4.85 (1H, m), 2.7 (4H, s), 2.3-2.6 (4H, m), 1.2 (4H, broad, disappears with $D_2O$).

*Example 26:*

*N-{2-[4-(6-N'-Methylaminohexyl)phenyl]-1-methylethyl}-2-hydroxy-2-(3-chlorophenyl)-ethanamine*

This was prepared in an identical manner to the compound described in Example 22 replacing 4-(2-N-formylaminoethyl)phenylpropan-2-one, by 4-(5-N-methylcarboxamidopentyl)phenylpropan-2-one. The intermediate carboxamide was reduced with lithium aluminium hydride using the same procedure as that given in Example 22. The title compound was isolated as the dihydrochloride salt as a 56:44 mixture of diastereoisomers, m.p. 185-200 (methanol/ethyl acetate).

τ ($CDCl_3$), free base: 8.8 (3H, d, J=8Hz), 8.2-8.8 (8H, m), 6.8-7.7 (15H, m), 5.4 (1H, m), 2.5-2.9 (8H, m).

*Example 27:*

*N-{2-[4-(4-N'-Methylaminobutyl)phenyl]-1-methylethyl}-2-hydroxy-2-(3-chlorophenyl)-ethanamine*

This was prepared in an identical manner to the compound described in example 22 replacing 4-(2-N-formylaminoethyl)phenylpropan-2-one by 4-(4-N-formylaminobutyl)phenylpropan-2-one. The title compound was isolated as the dihydrochloride salt as a 52:48 mixture of diastereoisomers, m.p. 113-117 (methanol/ethyl acetate).

τ ($d_6$ DMSO): 8.85 (3H, d, J=6Hz), 8.0-8.7 (4H, m), 6.3-7.7 (13H, m), 4.85 (1H, m), 3.6 (1H, broad, disappears with $D_2O$), 2.75 (4H, s), 2.35-2.6 (4H, m), 0.9 (3H, broad, disappears with $D_2O$), 0.25 (1H, broad, disappears with $D_2O$).

*Example 28:*

*N-[2-{4-[2-(R)-N'-Methylaminopropyl]phenyl}-1-methylethyl]-2-hydroxy-2-(3-chlorophenyl)ethanamine*

This was prepared in an identical manner to the compound described in example 22 replacing 4-[2-(N-formylamino)ethyl]phenylpropan-2-one by 4-[2-(R)-N-formylaminopropyl]phenyl-propropan-2-one. The title compound was isolated as the dihydrochloride salt as a 53:47 mixture of diastereo-isomers, m.p. 188-204 (methanol/ethyl acetate).

$[\alpha]^{25}_{D}$ $H_2O$ +1.9 $^1$HNMR identical to that of Example 25.

*Example 29:*

*N-{2-[4-(2-Methyl-2-N'-Methylaminopropyl)-phenyl]-1-methylethyl}-2-hydroxy-2-(3-chlorophenyl)ethanamine*

This was prepared in an identical manner to the compound described in Example 22 replacing

4-[2-(N-formylamino)ethyl]phenylpropan-2-one by 4-(2-methyl-2-N-formylaminopropyl)-phenylpropan-2-one. The title compound was isolated as the dihydrochloride salt as a 46:54 mixture of diastereo-isomers, m.p. 206-211 (methanol/ethyl acetate).

τ ($d_6$ DMSO): 8.8 (6H, s + 3H, d), 7.5 (3H, s), 6.3-7.2 (7H, m), 4.85 (1H, m), 3.6 (1H, broad, disappears with $D_2O$), 2.75 (4H, s), 2.4-2.65 (4H, m), 1.65 (4H, broad, disappears with $D_2O$).

*Example 30:*

*N-[2-(3-Methylamino-4-methylaminomethylphenyl)-1-methylethyl]-2-hydroxy-2-(3-chlorophenyl)ethanamine*

A solution of borane methyl sulphide (3 ml) in dry tetrahydrofuran (20 ml) was added dropwise at room temperature to a solution of N-[2-(3-methylamino-4-N'-methylcarboxamidophenyl)-1-methylethyl]-2-hydroxy-2-(3-chlorophenyl)-ethanamine (0.45 g) in dry tetrahydrofuran (20 ml), stirred for 20 min and then heated under reflux for 3.5 h. The solution was cooled and treated with a solution of methanol (15 ml) in dry tetrahydrofuran (20 ml) over 20 min. The mixture was then heated under reflux for a further 1.5 h cooled and the solvent removed. Chromatography of the residue on Kieselgel 60 using methanol/ammonia/chloroform (5:0.5:94.5) as eluent gave the title compound isolated as the trihydrochloride salt, m.p. 125-130 (ethyl acetate/methanol).

τ ($d_6$ DMSO): 8.8 (3H, d, J=6Hz), 7.3 (3H, s), 7.05 (3H, s), 6.1-6.9 (5H, m), 5.65 (2H, s), 4.85 (1H, m), 3.0-2.2 (7H, m), 1.2-2.1 (3H, broad), 0.4-1.2 (2H, broad), −0.2-0.4 (2H, broad).

*Example 31:*

*N-[2-(4-N'-Methylaminomethylphenyl)-1-methylethyl]-2-hydroxy-2-(3,4-dichlorophenyl)ethanamine*

N-[2-(4-N'-Methylcarboxamidophenyl)-1-methylethyl]-2-hydroxy-2-(3,4-dichlorophenyl)ethanamine (2,4 g) was dissolved in dry tetrahydrofuran (50 ml) under a nitrogen atmosphere and a solution of boranedimethyl sulphide (5 ml) in tetrahydrofuran (20 ml) was added dropwise over a period of 20 min. After completion of the addition the colourless solution was stirred for an additional 20 min and then heated under reflux for 4 h. The solution was cooled and excess borane destroyed by addition of methanol (15 ml) in tetrahydrofuran (30 ml) over a period of 30 min. The solution was heated under reflux for 1½ h, cooled and the diamine dihydrochloride precipitated by passage of hydrogen chloride through the solution. The product was filtered, washed with ether and dried to give the title compound (1.5 g, 65%), m.p. 253-270 (EtOAc/MeOH).

τ ($d_6$ DMSO): 8.85 (3H, d), 7,47 (3H, s), 6.3-7.4 (5H, m), 5.91 (2H, s), 4.82 (1H, m), 3.50 (1H, m, $D_2O$ exch), 2.20-2.70 (7H, m), 0.37 (4H, broad, $D_2O$ exch).

*Example 32:*

*N-{2-[4-(13-N'-Methylaminotridecyl)phenyl]-1-methylethyl}-2-hydroxy-2-(3-chlorophenyl)-ethanamine*

The title compound was made by an identical procedure to that described in Example 22, starting from 2-hydroxy-2-(3-chlorophenyl)ethanamine and 1-[4-(13-N-formylaminotridecyl)phenyl]-propan-2-one and isolated as the dihydrochloride salt, m.p. 135-145 (ethyl acetat/methanol).

τ ($d_6$ DMSO): 8.2-9.0 (22H, broad + 3H, d), 6.3-7.6 (9H, m + 3H, s), 4.7-5.1 (1H, m), 3.65 (1H, broad, disappears with $D_2O$), 2.4-2.9 (8H, m), 0.85 (3H, broad disappears with $D_2O$), 0.0 (1H, broad, disappears with $D_2O$).

*Derscription 1:*

*N-[2-(4-Cyanophenyl)-1-methylethyl]-2-hydroxy-2-phenylethanamine*

A mixture of 2-hydroxy-2-phenylethanamine (4.11 g) and (4-cyanophenyl)propan-2-one (4.77 g) was heated under reflux in benzene using a Dean and Stark apparatus for 4 h. The solvent was evaporated, replaced with methanol and sodium borohydride added portionwise. The solvent was removed, the residue partitioned between water and ether and the ether layer dried and evaporated. The residual oil was chromatographed on Kieselgel 60. Elution with 2% methanolchloroform gave the title compound 3.29 g, m.p. 110-121 (ethyl/acetate), isolated as a 23:77 mixture of diastereo-isomers.

τ ($CDCl_3$): 8.95 (3H, d, J=6Hz), 6.85-8.00 (7H, m, 2H disappears with $D_2O$), 5.4 (1H, dd), 2.8 (2H, d, J=8Hz), 2.65 (5H, s), 2.45 (2H, d, J=8Hz).

*Description 2:*

*N-{2-(4-N'-Methylcarboxamidophenyl)-1-methylethyl}-2-hydroxy-2-(2-benzofuranyl)-ethanamine*

A mixture of 2-benzofuranyl glyoxal (6.04 g) and 2-amino-1-(4-carbomethoxyphenyl) propane (6.7 g) in benzene (150 ml) was heated under reflux under Dean and Stark conditions until the theoretical amount of water had been removed. The benzene was evaporated, replaced with methanol and sodium borohydride (12 g) added portionwise. The methanol was removed, the residue partitioned between water and ether and the ether layers dried (magnesium sulphate) and evaporated to give an oil which was chromatographed on Silicagel. Elution with 2% methanolchloroform gave N-[2-(4-carbomethoxyphenyl)-1-methylethyl]-2-hydroxy-2-(2-benzofuranyl)-ethanamine.

This was dissolved in ethanolic methylamine (15 ml) and heated at 100° C for 5 h in an autoclave. The solvent was evaporated and the residue recrystallized to give N-[2-(4-N'-methylcarboxamidophenyl)-1-methylethyl]-2-hydroxy-2-(2-benzofuranyl)ethanamine as a mixture of diastereo-isomers, 5.4 g, m.p. 148-154° C (methanol/ethyl acetate).

τ ($d_6$ DMSO): 9.05 (3H, d, J=6Hz), 6.8-7.7 (5H, m + 3H, d, J=3Hz, collapses to singlet with $D_2O$), 5.6-6.7 (2H, broad, disappears with $D_2O$), 5.2 (1H, dd), 3.27 (1H, s), 2.6-2.9 (4H, m), 2.35-2.6 (2H, m), 2.2 (2H, d, J=8Hz), 1.62 (1H, q, J=3Hz, disappears with $D_2O$).

*Description 3:*

*N-[2-(4-N'N'-Dimethylcarboxamidophenyl)-1-methylethyl]-2-hydroxy-2-(3-trifluoromethylphenyl)ethanamine*

This was prepared in an identical manner to the compound described in Example 9 using 2-hydroxy-2-(3-trifluoromethylphenyl)ethanamine and (4-NN-dimethylcarboxamidophenyl)propan-2-one. The crude oil was chromatographed on Kieselgel 60. Elution with 10% methanolchloroform gave the title compound, isolated as the fumarate monohydrate, m.p. 153-158 (ethyl acetate).

τ ($d_6$ DMSO): 8.9 (3H, d, J=6 Hz), 7.02 (6H, s), 6.3-7.4 (5H, m), 4.9 (1H, m), 3.4 (2H, s), 2.6 (4H, m), 2.1-2.4 (4H, m), 0.3 (6H, broad, disappears with $D_2O$).

*Description 4:*

*N-[2-(4-N'-Methylcarboxamidophenyl)-1-methylethyl]-2-hydroxy-2-phenylethanamine*

This was prepared in an identical manner to the compound described in Example 9 using 2-hydroxy-2-phenylethanamine and (4-N-methylcarboxamidophenyl)propan-2-one.

τ ($CDCl_3$), 8.95 (3H, d, J=6Hz), 6.8-7.6 (5H, m), 7.05 (3H, d, J=5Hz, collapses to singlet with $D_2O$), 6.2 (2H, s, disappears with $D_2O$), 5.25 (1H, m), 3.3 (1H, q, J=5Hz, disappears with $D_2O$), 2.85 (2H, d, J=8Hz), 2.66 (5H, m), 2.32 (2H, d, J=8H).

*Description 5:*

*N-[2-(4-N'-Methylcarboxamidophenyl)-1-methylethyl]-2-hydroxy-2-(3-chlorophenyl)-ethanamine*

This was prepared in an identical manner to the compound described in Example 9 using 2-hydroxy-2-(3-chlorophenyl)ethanamine and (4-N-methylcarboxamidophenyl)propan-2-one. The crude oil was chromatographed on Kieselgel 60. Elution with 10% methanolchloroform gave the title compound as a 25:75 mixture of diastereoisomers, m.p. 68-76 (benzene).

τ ($d_6$ DMSO): 9.1 (3H, d, J=6Hz), 6.8-7.45 (5H, m + 3H, d, J=5H, collapses to singlet with $D_2O$), 5.45 (1H, m), 4.0-7.0 (2H, broad, disappears with $D_2O$), 2.85-2.65 (6H, m), 2.2 (2H, d, J=8Hz), 1.6 (1H, q, J=5Hz, disappears with $D_2O$).

*Description 6:*

*N-{2-[4-(2-N'-Methylcarboxamidoethyl)phenyl]-1-methylethyl}-2-hydroxy-2-(3-trifluoromethylphenyl)ethanamine*

N-[2-{4-[(E)-2-Carbomethoxyethenyl]phenyl}-1-methylethyl]oxy-2-hydroxy-2-(3-trifluoromethylphenyl)ethanamine (4.3 g) in tetrahydrofuran was added to a suspension of aluminium amalgam [from aluminium (2.8 g) and mercuric chloride (1.4 g)] in tetrahydrofuran at 0° C. The solution was left at this temperature for 1 h, allowed to warm to room temperature, filtered and the solvent removed under reduced pressure to give N-{2-[4-(2-carbomethoxyethyl)phenyl]-1-methylethyl}-2-hydroxy-2-(3-trifluoromethylphenyl)ethanamine. This was heated at 100° C for 5 h in an autoclave with ethanolic methylamine. The solution was evaporated and the residue chromatographed on Kieselgel 60. Elution with 8% methanolchloroform gave the title amide, m.p. 122-127 (benzene).

τ ($d_1$TFA): 8.9 (3H, d, J=6Hz), 6.8-7.7 (14H, m, 2H, disappears with $D_2O$), 5.35 (1H, dd), 4.32 (1H, broad, disappears with $D_2O$), 2.9 (4H, s), 2.3-2.6 (4H, m).

*Description 7:*

*N-[2-(4-N'-Acetylaminomethylphenyl)-1-methylethyl]-2-hydroxy-2-(3-trifluoromethylphenyl)ethanamine*

This was prepared in an identical manner to the compound escribed in Example 9 using 2-hydroxy-2-(3-trifluoromethylphenyl)ethanamine and (4-acetylaminomethylphenyl)propan-2-one. The crude oil was chromatographed on Kieselgel 60. Elution with 10% methanolchloroform gave the title compound, m.p. 68-104 (ether).

τ ($d_6$ DMSO): 9.1 (3H, d, J=6Hz), 8.1 (3H, s), 7.0-7.7 (5H, m), 5.8 (2H, d, J=6Hz), 5.32 (1H, m), 4.45 (1H, broad, disappears with $D_2O$), 2.8 (4H, s), 2.2-2.6 (4H, m), 1.7 (1H, t, J=6Hz, disappears with $D_2O$).

*Description 8:*

*(4-Acetylaminomethylphenyl)propan-2-one*

A mixture of ethylene glycol (4.36 g) and (4-cyanophenyl)propan-2-one (5.6 g) was heated for 18 h under reflux in benzene containing a trace of toxic acid. The reaction mixture was poured into water, the layers separated and the organic layer washed with sodium bicarbonate solution, dried ($MgSO_4$) and evaporated to give (4-cyanophenyl)propan-2-one, ethylene ketal (6.1 g). This was dissolved in tetrahydrofuran and the solution added to lithium aluminium hydride (2 g) in tetrahydrofuran. The solution was heated under reflux for 1 h at the end of addition. Water (2 ml), 2N sodium hydroxide (2 ml) followed by more water (6 ml) was added, the solution dried ($MgSO_4$), filtered and evaporated to give (4-aminomethylphenyl)propan-2-one, ethylene ketal (5.7 g).

τ ($CDCl_3$): 8.7 (3H, s), 8.1 (2H, broad s, disappears with $D_2O$), 7.1 (2H, s), 6.2 (4H, m + 2H, s), 2.7 (4H, s).

The above ketal (2.9 g) was dissolved in acetic anhydride and warmed on a steam bath for 0.5 h. Water (50 ml) was added and the solution heated for 0.75 h. The reaction mixture was extracted with

chloroform, the chloroform layers combined, washed with sodium bicarbonate solution (x3) and dried ($MgSO_4$) to give the title amide (3.0 g).

τ ($CDCl_3$): 8.0 (3H, s), 7.8 (3H, s), 6.3 (2H, s), 5.6 (2H, d, J=8Hz), 3.5 (1H, broad), 2.8 (4H, s).

*Description 9:*

*(4-Methylaminomethylphenyl)propan-2-one*

A mixture of (4-carbomethoxyphenyl)propan-2-one (20 g) and ethylene glycol (12.4 g) was heated for 18 h under reflux in benzene containing a trace of tosic acid. The reaction mixture was poured into water, the layers separated and the benzene layer washed with sodium bicarbonate solution, dried ($MgSO_4$) and evaporated to give the cyclic ethylene ketal. This was dissolved in methanolic methylamine and heated at 100° C for 5 h in an autoclave. Evaporation of the solvent gave 4-(N-methylcarboxamidophenyl)propan-2-one, ethylene ketal (30.31 g). This was dissolved in tetrahydrofuran and added dropwise to lithium aluminium hydride (5.0 g) in tetrahydrofuran. The solution was heated under reflux for 1 h at the end of addition. Water (5 ml), 2N sodium hydroxide (5 ml) followed by more water (15 ml) was added, the solution dried, filtered and evaporated to give the title compound protected as the ethylene ketal (23 g). The title compound was obtained by hydrolysis of the ketal in methanol-2N hydrochloric acid for 18 h at room temperature.

τ ($CDCl_3$): 7.95 (3H, s), 7.67 (3H, s), 7.6 (1H, broad), 6.4 (2H, s), 2.82 (4H, s).

*Description 10:*

*2-Hydroxy-2-(3-chloro-2-fluorophenyl)ethanamine*

2-Chloro-6-methylaniline (141.5 g, 1M) was dissolved in concentrated hydrochloric acid (325 ml), and water (450 ml); the stirred solution cooled to 0° C, and diazotized by the dropwise addition of a solution of sodium nitrite (69 g, 1M) in water (150 ml). The rate of addition was such that the temperature was kept below 5° C. After completion of the addition, the cold solution was stirred for a further ½ h and then filtered. Sodium tetrafluoroborate (110 g, 1.25M) was dissolved in water (60 ml), and the solution added slowly to the stirred diazonium solution, the temperature again being maintained below 5° C. The precipitate formed was filtered off after ½ h, washed successively with ice-water (150 ml), methanol (150 ml) and ether (200 ml). The colourless diazonium salt (207 g) was vacuum dried at room temperature, and then divided into four equal portions. Each portion was in turn placed in a round-bottomed flask attached to a condenser receiver flask and a trap containing sodium hydroxide solution. The flask was warmed with a Bunsen burner, and gradual decomposition of the diazonium salt effected over ¾ h. The combined distillates were dissolved in ether, washed with 2N sodium hydroxide solution and brine, and dried ($MgSO_4$). The solvent was evaporated and the residual oil distilled at water-pump pressure. Collection of the fraction b.p. 74-77° C gave 3-chloro-2-fluorotoluene (105.4 g, 73%) as a colourless oil.

τ ($CCl_4$): 2.5-3.2 (m, 3H); 7.7 (s, 3H).

3-Chloro-2-fluorotoluene (105.4 g, 0.73M) was dissolved in carbon tetrachloride (1 l), and N-bromosuccinimide (130 g, 0.73M) and benzoyl peroxide (1 g) added. The mixture was stirred and heated to reflux for 18 h with illumination by a 100 W tungsten-filament bulb. The mixture was then cooled, filtered and the solvent evaporated. The residue was distilled at 0.6 mm pressure, collection of the fraction b.p. 76-78° C affording 3-chloro-2-fluorobenzyl bromide (108.6 g, 67%) as a colourless oil.

τ ($CCl_4$): 2.5-3.15 (m, 3H); 5.55 (s, 2H).

3-Chloro-2-fluorobenzyl bromide (108.6 g, 0.486M) was dissolved in methanol (100 ml) and added to a stirred solution of sodium (12.4 g, 0.54M) in methanol (400 ml) containing 2-nitropropane (48 ml, 0.54M). After completion of the addition, the reaction mixture was heated to reflux for 1 h, cooled and the solvent evaporated. The residue was extracted with ether and water, the aqueous layer discaded, and the ethereal layer washed with 2N sodium hydroxide solution and water until the washing were neutral. The solution was dried ($MgSO_4$) and the solvent evaporated. The orange residue was distilled at 3 mm pressure, collection of the fraction b.p. 60-62° C affording 3-chloro-2-fluorobenzaldehyde (47.0 g, 61%) as a colourless oil which solidified on standing in the cold.

τ ($CCl_4$): −0.4 (s, 1H), 2.1-2.6 (m, 3H).

To a stirred solution of 3-chloro-2-fluorobenzaldehyde (43 g, 0.27M) in dry ether (200 ml) was added dropwise trimethylsilyl cyanide (40 ml, 0.3M) in the presence of a little zinc iodide. The mixture was stirred for 2 h and then left overnight. The solution was then transferred to a pressure-equalizing dropping funnel and added dropwise to a slurry of lithium aluminium hydride (11.4 g, 0.3M) in dry ether (300 ml), kept cool in an ice bath. After completion of the addition, the mixture was heated to reflux for 1 h, then cooled and quenched by adding water (11.4 ml), 2N sodium hydroxide (11.4 ml) and water (34.2 ml). The reaction mixture was filtered, the solids washed well with chloroform, and the filtrate washed with water and dried ($MgSO_4$). The solvents were evaporated, and the residue crystallized (40 g). Recrystallization from ether afforded 2-hydroxy-2-(3-chloro-2-fluorophenyl)ethanamine as colourless crystals (19.2 g, 37%), m.p. 80-81° C.

τ ($CDCl_3$): 2.3-3.0 (m, 3H); 5.0 (dd, 1H); 6.8-7.5 (m, 5H).

*Description 11:*

*4-(2-N-Formylaminoethyl)phenylpropan-2-one*

2-Chloropropionyl chloride (29.6 ml) in 1,2-dichloroethane was added dropwise to a mixture of aluminium chloride (60.8 g) and 2-phenethylamine formamide (45.4 g) in 1,2-dichloroethane at ice-bath temperature. The solution was heated

under reflux for 2 h, poured into ice and the organic layer separated and dried. Evaporation of the solvent gave an oil which was chromatographed on Kieselgel 60. Elution with chloroform gave 4-(2-chloropropionyl)phenethylamine formamide, 49.0 g.

τ ($CDCl_3$): 8.3 (3H, d, J=6Hz), 7.1 (2H, t, J=6Hz), 6.15-6.7 (2H, m), 4.75 (1H, q, J=6Hz), 3.45 (1H, broad), 2.65 (2H, d, J=8Hz), 2.05 (2H, d, J=8Hz), 1.9 (1H, s).

Potassium acetate (96.8 g), glacial acetic acid (29.2 ml) and potassium iodide (50.7 g) were added to the above formamide (39.3 g) in acetone (800 ml). The reaction mixture was stirred vigorously and heated under reflux for 18 h. Water was added to the cool solution, the solvent evaporated and the residue partitioned between water and dichloromethane. The organic layers were washed with sodium bicarbonate solution, dried and evaporated to give 4-(2-acetoxypropionyl)phenethylamine formamide (44.7 g) as an oil which was not purified further.

τ ($CDCl_3$): 8.45 (3H, d, J=7Hz), 7.83 (3H, s), 7.1 (2H, t, J=6Hz), 6.2-6.8 (2H, m), 4.05 (1H, q, J=7Hz), 3.35 (1H, broad), 2.65 (2H, d, J=8Hz), 2.15 (2H, d, J=8Hz), 1.95 (1H, s).

Sodium borohydride (4.28 g) was added portionwise to the above acetate (44.7 g) in ethanol. After addition the solvent was evaporated, the residue taken up in water and extracted with ethyl acetate and chloroform. The combined organic layers were dried and evaporated to give the hydroxyacetate (16.98 g) which was heated with potassium bisulphate (14.09 g) at 80-100°C/2mm until the mixture darkened. The residue was partitioned between water and chloroform. The organic layer was separated, dried and evaporated to give an oil (7.47 g), which was chromatographed in Kieselgel 60. Elution with 2% methanolchloroform gave the title compound (1.7 g).

τ ($CDCl_3$): 7.85 (3H, s), 7.25 (2H, t, J=6Hz), 6.35-6.75 (2H, m), 6.3 (2H, s), 2.85 (4H, s, + 1H, broad), 2.1 (1H, s).

*Description 12:*

*4-[1-(S)-N-Formylaminoethyl]phenylpropan-2-one*

The above compound was prepared in an identical manner to that described in Description 11 starting from (S)-α-methylbenzylamine formamide.

τ ($CDCl_3$): 8.45 (3H, d, J=6Hz), 7.8 (3H, s), 6.3 (2H, s), 4.75 (1H, q, J=6Hz), 2.7-2.9 (4H, m + 1H, broad), 1.95 (1H, s).

*Description 13:*

*4-[-(R)-N-Formylaminoethyl]phenylpropan-2-one*

The above compound was prepared in an identical manner to that described in Description 11 starting from (R)-α-methylbenzylamine formamide. $^1$HNMR identical to that of Description 12.

*Description 14:*

*4-(2-N-Formylaminopropyl)phenylpropan-2-one*

The above compound was prepared in an identical manner to that described in Description 11 starting from α-methylphenylamine formamide.

τ ($CDCl_3$): 8.9 (3H, d, J=6Hz), 7.85 (3H, s), 7.2 (2H, m), 6.35 (2H, s), 5.7 (1H, m), 3.25 (1H, broad), 2.9 (4H, s), 2.1 (1H, s).

*Description 15*

*4-(5-N-Methylcarboxamidopenthyl)phenylpropan-2-one*

Aluminium chloride (27.5 g) was added, portionwise, to a stirred, ice-cooled solution of 6-bromohexanoic acid (20 g) in dry benzene (150 ml). The mixture was heated under reflux for 2 h and stirred at ambient temperature for 16 h. The reaction mixture was poured on to ice-water. The excess benzene was evaporated and the aqueous residue was extracted into ether. The ether extract was dried ($MgSO_4$) and evaporated to give 6-phenylhexanoic acid (17.5 g) as an oil.

τ ($CDCl_3$): 8.0-8.9 (6H, m), 7.2-7.9 (4H, m), 2.4-3.0 (5H, m).

6-Phenylhexanoic acid (15 g) was added to dry thionyl chloride (25 ml) with stirring and cooling. The mixture was heated at 80° C for 1 h, cooled and evaporated to a brown oil. Methylamine gas was bubbled into a solution of the acid chloride in dry ether. The resulting solution was concentrated to give N-methyl-6-phenyl hexanamide as an oil which crystallized after chromatography on Kieselgel 60 with chloroform as eluent.

τ ($CHCl_3$); 8.2-8.8 (6H, m), 7.65-8.1 (2H, m), 7.1-7.5 (3H, d, J=8Hz plus 2H, m), 4.3 (1H, broad), 2.6-3.0 (5H, s).

2-Chloropropionyl chloride (5 g) was added, dropwise, under nitrogen, to a stirred slurry of aluminium chloride (5.5 g) in 1,2-dichloroethane (80 ml). The mixture was stirred at ambient temperature for 15 min. N-Methyl-6-phenyl hexanamide (8 g) in 1,2-dichloroethane (10 ml) was added dropwise, under nitrogen, to the stirred solution. The reaction mixture was heated under reflux for 1 h, left at ambient temperature for 24 h, poured onto ice-water, the organic layer separated, washed with water and dried. Evaporation of the solvent gave an oil which was chromatographed on Kieselgel 60 using chloroform as eluent to give 2-chloro-1-[4-(5-N-methylcarboxamido)pentyl]phenylpropan-1-one (3.73 g).

τ ($CDCl_3$): 8.2-8.8 (3H, d, J=8Hz plus 6H, m), 7.6-8.05 (2H, m), 7.1-7.5 (3H, d, J=8Hz plus 2H, m), 4.75 (1H, q, J=6Hz), 4.3 (1H, broad), 2.7 (2H, d, J=8Hz), 2.05 (2H, d, J=8Hz).

A mixture of the above α-chloroketon (3.73 g) and anhydrous potassium acetate (2 g) in acetone (80 ml) was stirred and heated under reflux for 24 h). The cooled reaction mixture was diluted with water (80 ml), the solvent was evaporated

and the aqueous residue extracted into dichloromethane. The organic layer was washed with sodium bicarbonate solution, dried ($MgSO_4$) and evaporated to give 2-acetoxy-1-{4-[5-(N-methylcarboxamido)penthyl]phenyl}propan-1-one (3.27 g).

τ ($CDCl_3$): 8.2-8.8 (3H, d, J=8Hz plus 6H, m), 7.7-8.05 (3H, s plus 2H, m), 7.2-7.55 (3H, d, J=8Hz plus 2H, m), 4.1 (1H, q, J=8Hz), 3.65 (1H, broad), 2.75 (2H, d, J=8Hz), 2.1 (2H, d, J=8Hz).

Excess sodium borohydride (2.75 g) was added, portionwise, to an ice-cooled solution of the above α-ketoacetate (3.27 g) in ethanol. After stirring at ambient temperature for 2 h the solvent was evaporated and the residue partitioned between water and chloroform. The organic layer was dried and evaporated to give the 1,2-diol (2.27 g) which was taken up in dry toluene (80 ml), treated with p-toluene sulphonic acid (0.5 g) and stirred and heated under reflux, with water removal, for 8 h. The solvent was evaporated and the residue partitioned between water and dichloromethane. The organic layer was washed with sodium bicarbonate solution, dried ($MgSO_4$) and evaporated to give an oil which was chromatographed on Kieselgel 60. Elution with chloroform gave the title compound (1.22 g).

τ ($CDCl_3$): 8.2-8.8 (6H, m), 7.7-8.0 (3H, s plus 2H, m), 7.15-7.50 (3H, d, J=8Hz plus 2H, m), 6.35 (2H, s), 3.5 (1H, broad), 2.8 (4H, s).

*Description 16:*

*4-(4-N-Formylaminobutyl)phenylpropan-2-one*

The above compound was prepared in an identical manner to that described in Description 11 starting from 4-phenylbutylamine formamide.

τ ($CDCl_3$: 8.1-8.6 (4H, m), 7.85 (3H, s), 7.4 (2H, m), 6.75 (2H, m), 6.35 (2H, s), 3.3 (1H, broad), 2.87 (4H, s), 1.9 (1H, s).

*Description 17:*

*4-[2-(R)-N-Formylaminopropyl]phenylpropan-2-one*

The above compound was prepared in an identical manner to that described in Description 11 starting from (R)-α-methylphenethylamine formamide. $^1$HNMR identical to that of Description 14.

*Description 18:*

*4-(2-Methyl-2-N-formylaminopropyl)phenylpropan-2-one*

Starting from α,α-dimethylphenethylamine formamide the above compound was prepared in a similar manner to that described in Description 11 except that the final dehydration step of the diol was accomplished using 4-toluenesulphonic acid in xylene at reflux with water removal.

τ ($CDCl_3$): 8.6 (6H, s), 7.8 (3H, s), ((6.95 (s) + 7.25 (s), total 2H), 6.35 (2H, s), 4.6 (1H, broad), 2.85 (4H, s), 2.05 (1H, d, J=2Hz).

*Description 19:*

*N-[2-(3-Methylamino-4-N'-methylcarboxamidophenyl)-1-methylethyl]-2-hydroxy-2-(3-chlorophenyl)ethanamine*

A solution of 2-fluoro-4-methylaniline (18.5 g) in 25% aqueous hydrochloric acid at 0° C was treated with a solution of sodium nitrite (10.35 g) in water (50 ml) over 20 min. The mixture was stirred at 0° C for 20 min then neutralized with solid potassium acetate, filtered and added dropwise to a stirred solution of copper (1) cyanide (13.35 g)/potassium cyanide (24.37 g) in water (125 ml) at 70° C. The reaction was stirred at this temperature for 20 min then steam distilled. The distillate was extracted with ether (3 × 100 ml), dried (magnesium sulphate) and evaporated to give 2-fluoro-4-methylbenzonitrile (69-70° C/2.0 mm).

τ ($CDCl_3$): 7.6 (3H, s), 3.1 (1H, d), 2.9 (1H, s), 2.55 (1H, t).

A solution of this nitrite (11 g) in 2N sodium hydroxide (150 ml) was heated under reflux for 2.5 h, cooled and acidified with concentrated hydrochloric acid. The precipitate was filtered, washed with water (2 × 50 ml) and dried to give 2-fluoro-4-methylbenzoic acid, m.p. 163-166° C (benzene).

τ ($d_6$ DMSO): 7.7 (3H, s), 3.0 (1H, s), 2.8 (1H, broad, s), 2.25 (1H, t), −3.0 (1H, broad).

The above acid (18 g) was esterified by heating in methanol containing sulphuric acid (2 ml) under reflux for 18 h. The reaction mixture was cooled, poured onto ice and the organic layer extracted into ether, washed with potassium bicarbonate solution and dried. Evaporation gave the methyl ester, m.p. 52-54° C.

τ ($CDCl_3$): 7.7 (3H, s), 6.1 (3H, s), 3.18 (1H, d), 3.0 (1H, s), 2.25 (1H, t).

The above ester (12 g) in carbon tetrachloride was treated with N-bromosuccinimide (13.35 g) and benzoyl peroxide (0.25 g) and heated under reflux for 3.5 h with light irradiation. The reaction mixture was cooled, filtered and the solvent removed to give 3-fluoro-4-carbomethoxybenzyl bromide, m.p. 80-81° C (carbon tetrachloride/petrol).

τ ($CDCl_3$): 7.6 (2H, s), 6.1 (3H, s), 3.0 (2H, m), 2.2 (1H, m).

A solution of the above bromide (19 g) in methanol (75 ml) was added dropwise to a solution of 2-nitropropane (7.56 g) and sodium (1.84 g) in methanol (75 ml) at room temperature. After the addition was complete the mixture was heated on a steam bath for 1 h, cooled and the solvent evaporated. The residue was dissolved in ether and washed with water (100 ml), 2N sodium hydroxide (75 ml) and more water. The organic layer was dried, evaporated and distilled to give 3-fluoro-4-carbomethoxybenzaldehyde (95.98/1.0 mm).

τ ($CDCl_3$): 6.0 (3H, s), 2.3 (2H, m), 1.8 (1H, t), −0.1 (1H, s).

A mixture of the above aldehyde (2.4 g) and n-butylamine (1.1 g) in benzene (50 ml) was heated

under reflux for 2 h using a Dean and Stark head. The benzene was removed, replaced with glacial acetic acid and nitroethane (2 g) added. The mixture was heated at 100° C for 1 h, cooled diluted with water. The precipitated 1-(3-fluoro-4-carbomethoxyphenyl)-2-nitroprop-1-ene, m.p. 110-112° C, was filtered and dried.

τ ($d_6$ DMSO): 7.6 (3H, s), 6.1 (3H, s), 2.2 (4H, m).

Glacial acetic acid (20 ml) was added over 20 min to a mixture of the above nitropropene (1.5 g), iron powder (3.4 g) and water (2.5 ml) in methanol (20 ml) at reflux. The mixture was heated under reflux for 3 h, cooled, filtered through celite and the filtrate extracted with dichloromethane, dried, evaporated and distilled to give (3-fluoro-4-carbomethoxy)phenylpropan-2-one (60-63° C/1 mm).

τ ($CDCl_3$): 7.8 (3H, s), 6.2 (2H, s), 6.05 (3H, s), 3.05 (1H, d), 2.85 (1H, s), 2.1 (1H, t).

A mixture of 2-hydroxy-2-(3-chlorophenyl)-ethanamine (0.79 g) and (3-fluoro-4-carbomethoxyphenyl)propan-2-one (0.9 g) in benzene (50 ml) was heated under reflux for 3 h using a Dean and Stark head. The solvent was removed, replaced with methanol and sodium borohydride (0.2 g) was added portionwise. After stirring for 1 h the solvent was evaporated, the residue partitioned between ethyl acetate and water and the organic layer dried. Removal of the solvent and chromatography on Kieselgel 60 using 2% methanolchloroform as eluent gave N-[2-(3-fluoro-4-carbomethoxyphenyl)-1-methylethyl]-2-hydroxy-2-(3-chlorophenyl)ethanamine, m.p. 95-100° C (ether/hexane).

τ ($CDCl_3$): 8.9 (3H, d, J=6Hz), 8.0-6.9 (7H, m), 6.03 (3H, s), 5.35 (1H, dd), 3.1-2.6 (6H, m), 2.1 (1H, t).

A solution of the above ethanamine (1.0 g) in ethanolic methylamine (25 ml) was placed in a stainless steel pressure vessel and heated at 110° C for 5 h, cooled and the solvent removed. Chromatography on Kieselgel 60 using methanol/ammonia/chloroform (6:1:93) as eluent gave the title compound, m.p. 90-100° C.

τ ($d_6$ DMSO): 9.1 (3H, d, J=6Hz), 8.4 (1H, broad s), 7.5-7.0 (11H, m), 6.7 (1H, bs), 5.4 (1H, m), 4.65 (1H, bd), 3.6 (2H, m), 3.0-2.4 (4H, m), 2.2 (1H, bd), 1.8 (1H, bd).

*Description 20:*

*N-[2-(4-N'-Methylcarboxamidophenyl)-1-methylethyl]-2-hydroxy-2-(3,4-dichlorophenyl)ethanamine*

N-[2-(4-Carbomethoxyphenyl)-1-methylethyl]-2-hydroxy-2-(3,4-dichlorophenyl)ethanamine (3.0 g) was dissolved in 33% methylamine in ethanol solution (20 ml) and heated to 100° C in a pressure vessel for 5 h. The solvent was evaporated and the residue crystallized from ethyl/ethyl acetate to give the title amide (2.4 g, 80%).

τ ($d_6$ DMSO): 9.08 (3H, d), 6.9-7.6 (8H, m), 6.10 (2H, broad, $D_2O$ exch), 5.38 (1H, m), 2.1-2.9 (7H, m), 1.62 (1H, m, $D_2O$ exch).

*Description 21:*

*1-[4-(13-N-Formylaminotridecyl)phenyl]propan-2-one*

n-Butyl lithium (75 ml of 1.55M solution in hexane) was added, dropwise, under nitrogen to a solution of phenyl acetylene (10.2 g, 0.1M) in dry tetrahydrofuran (150 ml) at −10° C. The solution was stirred at −10° C for 45 min and added, dropwise, to a stirred solution of 1,10-dibromodecane (75 g, 0.25M) in a mixture of dry tetrahydrofuran (200 ml) and dry hexamethylphosphoramide (100 ml). The mixture was stirred at ambient temperature for 18 h, poured into ice-water and extracted with n-hexane. The organic layer was washed with water, dried (magnesium sulphate) and evaporated to a brown oil. Excess 1,10-dibromodecane was removed by distillation under reduced pressure (130-136°C/1.5 mmHg). The residual alkyne was hydrogenated at ambient temperature and pressure, in ethanol, over palladium on barium sulphate until the theoretical volume of hydrogen had been taken up. The catalyst was filtered and the filtrate evaporated to give 1-bromo-12-phenyldodecane as an oil.

τ ($CDCl_3$): 8.0-8.9 (20H, broad), 7.25-7.55 (2H, m), 6.5-6.8 (2H, t), 2.6-3.0 (5H, broad s).

1-Bromo-12-phenyldodecane (16 g) was added, in portions, to a stirred slurry of sodium cyanide (3 g) in dimethylsulphoxide (20 ml) at 80-90° C. The mixture was stirred and heated at 100° C for 2 h, cooled, diluted with water (200 ml) and extracted with dichloromethane (2 × 200 ml). The organic layer was washed with sodium chloride, dried (magnesium sulphate) and evaporated to give 1-cyano-12-phenyldodecane as an oil. The crude nitrile (12.85 g) in dry ether (30 ml) was added, dropwise, under nitrogen, to a stirred slurry of lithium aluminium hydride (3.8 g) in dry ether (100 ml). The mixture was stirred and heated under reflux for 2 h, cooled, treated with water (3.8 ml), 2N sodium hydroxide solution (3.8 ml) and water (14.4 ml) and filtered. The filtered solid was washed with chloroform. The combined filtrates were evaporated to give 1-amino-13-phenyltridecane as an oil. The amine (12 g) in benzene (150 ml) was treated with excess formic acid (25 ml) and stirred and heated under reflux, with water-separation for 4 d. The solvent was evaporated and the residue purified by column chromatography on Kieselgel 60 using dichloromethane as eluent to give 1-N-formylamino-13-phenyltridecane as a waxy solid.

τ ($CDCl_3$): 8.4-8.8 (22H, broad s), 7.2-7.5 (2H, m), 6.5-7.0 (2H, m), 4.0 (1H, broad, disappears with $D_2O$), 2.75 (5H, s), 1.8 (1H, s).

1-[4-(13-N-Formylaminotridecyl)phenyl]propan-2-one was prepared from 1-N-formylamino-13-phenyltridecane in an identical manner to that described in Description 11.

τ ($CDCl_3$): 8.3-8.9 (22H, broad s), 7.85 (3H, s), 7.2-7.55 (2H, m), 6.5-6.9 (2H, m), 6.3 (2H, s), 3.8 (1H, broad, disappears with $D_2O$), 2.7-2.9 (4H, m), 1.85 (1H, broad s).

*Demonstration of Effectiveness of Compounds*

I *Antiobesity Activity*

The compounds were administered by oral gavage in water or carboxymethylcellulose suspension to genetically obese mice daily for 28 d. At the end of the time the caracass composition was determined. The results obtained were as follows:

| Compounds of Example No. | Dose (mg/kg *p.o.*) | Lipid/Mouse (g) | |
|---|---|---|---|
| | | Treated | Control |
| 1 | 6.25 | 17.98 | 21.52 |
| 2 | 11.8 | 20.87 | 25.09 |
| 3 | 12.2 | 18.56 | 20.98 |
| 6 | 10.9 | 17.38 | 18.75 |
| 7 | 9.9 | 20.56 | 23.13 |
| 9 | 10.8 | 20.63 | 22.95 |
| 12 | 12.5 | 20.38 | 24.60 |
| 16 | 11.3 | 16.03 | 22.55 |

II *Effect on Energy Expenditure*

The effect of the compounds on the energy expenditure of mice was demonstrated by means of the following procedure—

Female CFLP mice each weighing approximately 24 g, were given food and water *ad lib* before and during the experiment. The compounds were dissolved in water by addition of 1 mol of hydrochloric acid per mole of compound and these solutions were administered orally to each of 12 mice. A further 12 mice were dosed orally with water. The mice were placed in boxes through which air was drawn and the oxygen content of the air leaving the boxes was measured. The energy expenditure of the mice was calculated for 21 h after dosing from the volume of air leaving the boxes and its oxygen content, following the principles described by J.B. de V. Weir, "J. Physiol." (London), *109*, 1-9 (1949). The food intake of the mice was measured over this same period of 21 h. The results are expressed as a percentage of the mean food intake or rate of energy expenditure of the mice dosed with water.

| Compound of Example No. | Dose (mg/kg *p.o.*) | Mean Energy Expenditure | | Mean Food Intake (%) |
|---|---|---|---|---|
| | | 0-3 h | 0-21 h | |
| 1 | 19.8 | 188 | 126 | 103 |
| 2 | 23.6 | 158 | 125 | 79 |
| 3 | 24.5 | 156 | 122 | 140 |
| 4 | 25.2 | 126 | 112 | 82 |
| 5 | 22.9 | 131 | 105 | 82.5 |
| 6 | 21.7 | 152 | 125 | 73 |
| 7 | 19.8 | 154 | 117 | 90 |
| 8 | 22.6 | 193 | 140 | 75 |
| 9 | 21.7 | 137 | 117 | 70 |
| 10 | 22.2 | 92 | 101 | 102 |
| 11 | 21.7 | 132 | 108 | 115 |
| 12 | 25.1 | 147 | 123 | 97 |
| 13 | 25.2 | 124 | 111 | 86 |
| 14 | 26.0 | 166 | 126 | 90 |
| 15 | 24.5 | 131 | 115 | 73 |
| 16 | 5.6 | 136 | 121 | 85 |
| 17 | 2.8 | 147 | 126 | 91 |
| 18 | 24.1 | 118 | 107 | 101 |
| 19 | 23.7 | 126 | 106 | 50 |
| 20 | 24.8 | 132 | 110 | 87 |
| 21 | 23.6 | 160 | 133 | 82 |
| 22 | 28.3 | 148 | 132 | 114 |
| 23 | 28.3 | 103 | 98 | 95 |
| 24 | 28.3 | 113 | 103 | 96 |
| 25 | 29.1 | 154 | 125 | 78 |
| 26 | 26.4 | 164 | 141 | 84 |
| 27 | 24.9 | 175 | 134 | 56 |
| 28 | 24.1 | 131 | 104 | 80 |
| 29 | 24.9 | 123 | 106 | 88 |
| 30 | 26.2 | 121 | 101 | 78 |

III *Cardiac Activity*

Rat hearts were perfused by the Langendorff procedure.

Hearts were dissected free within 30 s of death and reverse perfused *via* the aorta and coronary vessels with Krebs-Ringer bicarbonate solution (pH 7.4, 37° C) gassed with 95% oxygen: 5% carbon dioxide at a flow rate between 8-12 $cm^3$/min. Responses were observed after injection of drug dissolved in isotonic saline into the perfusion media. Heart rate and tension were displayed on an Ormed MX2P recorder *via* a tension transducer and heart ratemeter.

Results are expressed as a percentage of the maximum reponse due to salbutamol.

| Compound of Example No. | Dose added to Perfusate (μg) | Heart Tension | Heart Rate |
|---|---|---|---|
| Salbutamol | — | 100 | 100 |
| 1 | 30 | 18 | 14 |
| 2 | 30 | 8 | 6 |
| 3 | 30 | 28 | 0 |
| 4 | 30 | 3 | 6.5 |
| 5 | 30 | 18 | 0 |
| 6 | 30 | 0 | 0 |
| 7 | 10 | 0 | 25 |
| 8 | 30 | 13 | 8 |
| 9 | 10 | 33 | 15 |
| 10 | 10 | 33 | 0 |
| 11 | 30 | 0 | 6 |
| 12 | 30 | 0 | 13 |
| 13 | 30 | 0 | 0 |
| 14 | 30 | 12 | 0 |
| 15 | 30 | 0 | 0 |
| 16 | 30 | 0 | 0 |
| 17 | 30 | 0 | 0 |
| 21 | 30 | 0 | 0 |
| 22 | 30 | 100 | 43 |
| 25 | 30 | 33 | 39 |
| 26 | 30 | 10 | 33 |
| 27 | 30 | 25 | 55 |

IV *Hypoglycaemic Activity*

Female CFLP mice, weighing approx. 25 g, were fasted for 24 h prior to the study. The compounds under study were administered orally as an aqueous solution to each of 6 mice. 30 min later a blood sample (20 $\mu m^3$) was obtained from the tail for the analysis of blood glucose. Immediately after taking the blood sample, glucose (1 g/kg body weight) was administered subcutaneously to each mouse. 6 mice were given water as a control. Blood samples were then obtained from each mouse at 30 min intervals for 120 min.

Compounds that produced a significant ($P<0.05$) reduction on blood glucose, compared with control mice given water, at any time interval, were considered active. The area under the blood curve over the 2 h period after the administration of the glucose was calculated for each compound and compared with the value for control animals.

| Compound of Example No. | Dose (μmol/kg) | Reduction in Area under Blood Glucose Curve (%) |
|---|---|---|
| 1 | 2.5 | 29 |
| 2 | 2.5 | 24 |
| 4 | 12.5 | 31 |
| 8 | 12.5 | 52 |
| 10 | 100.0 | 49 |
| 12 | 2.5 | 37 |
| 14 | 12.5 | 46 |
| 15 | 2.5 | 19 |
| 16 | 2.5 | 33 |
| 23 | 50 | 26 |
| 24 | 50 | 17 |
| 25 | 2.5 | 41 |
| 26 | 2.5 | 53 |
| 27 | 2.5 | 10 |

V *Anti-Inflammatory Activity*

The method used is based on that described by G. Tonelli *et al.*, "Endocrinology", *77*, 625-634, 1965. An inflammation is induced in the rat ear by the application of 50 µl of a 1% solution of croton oil in tetrahydrofuran, test compounds being dissolved in the irritant vehicle. After 6 h the inflammation is assessed by killing the animals and weighing the ears. Topical anti-inflammatory activity of a compound is generally considered to be shown when a significant (15% level) reduction in ear weight is seen compared to non-drug treated control.

| Compound of Example No. | Dose (mg/Rat ear) | Activity |
|---|---|---|
| 2 | 0.5 | Active |
| 8 | 2 | Active |

VI *Platelet Aggregation Inhibition Activity*

Male CFLP mice (ca. 20 g, n=11 or 12) were dosed orally with compound or vehicle (controls) after an overnight fast. 2 h later each mouse received an intravenous dose of collagen (400 µg/kg, pH 6-6.6). Exactly 30 s after injection of collagen, each mouse was placed in a chamber of $CO_2$ until respiration ceased. Blood platelet concentration was determined (Ultra.Flo 100 whole blood platelet counter, Clay Adams) in blood samples (3 µl) taken immediately from the inferior vena cava. Each concentration was expressed as a per cent of that obtained in a tail blood sample taken immediately before injection of collagen. Results are given in the table below.

| Compound | Dose *p.o.* (µmol (mg)/kg) | % Inhibition of response to Collagen |
|---|---|---|
| Aspirin | 600(108) | 29 $p<0.05$ |
| 2 | 10(4.25) | 29 $p<0.01$ |
| 3 | 10(4.39) | 25 $p<0.02$ |
| 8 | 10(4.06) | 37 $p<0.001$ |

**Claims**

1. A compound of Formula (I):

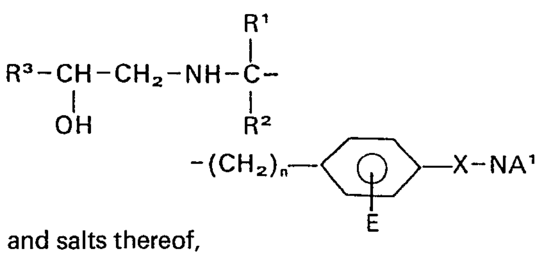

(I)

and salts thereof,
wherein
$R^1$ is hydrogen or methyl;
$R^2$ is hydrogen or methyl;
$R^3$ is phenyl optionally substituted with one or two of the following: fluorine, chlorine, bromine, trifluoromethyl or $C_{1-6}$ alkyl; or is benzofuran-2-yl;
n is 1 or 2;
X is $C_{1-15}$ straight or branched alkylene;
$A^1$ is hydrogen, $C_{1-6}$ alkyl, or optionally substituted benzyl;
$A^2$ is hydrogen, $C_{1-6}$ alkyl, or optionally substituted benzyl; and
E is hydogen, halogen, $C_{1-6}$ alkoxy, $C_{1-6}$ alkyl or mono- or di-$C_{1-6}$ alkylamino.

2. A compound according to Claim 1, in which $R^3$ is phenyl optionally substituted by one of the following: fluorine, chlorine, bromine or trifluoromethyl; $X_1$ is $C_{1-6}$ straight or branched alkylene; $A^1$ is hydrogen or $C_{1-6}$ alkyl; $A^2$ is hydrogen or $C_{1-6}$ alkyl; and E is hydrogen.

3. A compound according to Claim 1 or 2, in which $A^1$ and $A^2$ are simultaneously hydrogen, or one represents hydrogen and the other represents methyl.

4. A compound according to any one of Claims 1 to 3, in which X represents methylene.

5. A process for producing a compound of Formula (I), as defined in Claim 1, which comprises reducing a compound of Formula (II);

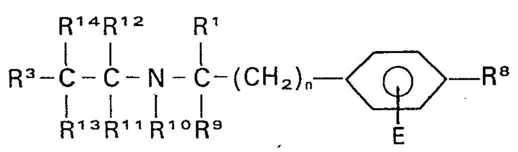

(II)

wherein $R^1$, $R^2$, E and n are as defined in relation to Formula (I), $R^8$ is a group $-X-NA^1A^2$ or $-Y-Z$, wherein $A^1$, $A^2$ and X are as defined in relation to Formula (I) and Y is a bond or $C_{1-14}$ straight or branched alkylene, and Z is cyano or

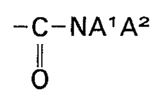

or Y is $C_{1-15}$ straight or branched alkylene and Z is

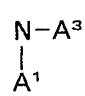

wherein $A^3$ is $C_{1-5}$ alkyl carbonyl, $R^9$ is a group $R^2$ as defined in relation to Formula (I) or together with $R^{10}$ forms a bond; $R^{10}$ is hydrogen, benzyl or

together with $R^9$ or $R^{11}$ forms a bond; $R^{11}$ is hydrogen or together with $R^{12}$ forms a bond; $R^{12}$ is hydrogen or together with $R^{11}$ forms an oxo-group; $R^{13}$ is hydroxyl or together with $R^{14}$ forms an oxo-group; $R^{14}$ is hydrogen or together with $R^{13}$ forms an oxo-group, provided that there is no more than one oxo-group and no more than one bond represented by any of $R^9$ to $R^{14}$ and optionally thereafter forming a salt, of the compound of Formula (I) so formed and/or converting the compound of Formula (I) so formed into a further compound of Formula (I).

6. A process for producing a compound of Formula (I), as defined in Claim 1, which comprises reacting a compound of Formula (III):

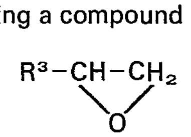

(III)

with a compound of Formula (IV):

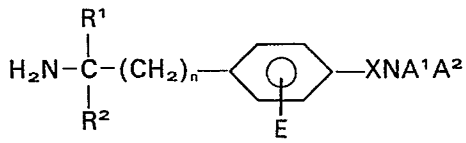

(IV)

in which $R^1$, $R^2$, $R^3$, E, $A^1$, $A^2$, X and n are as defined in relation to Formula (I), and optionally thereafter forming a salt of the compound of Formula (I) so formed and/or converting the compound of Formula (I) so formed into a further compound of Formula (I).

7. A pharmaceutical composition comprising a compound of Formula (I), as defined in any one of Claims 1 to 4 or a pharmaceutically acceptable salt thereof, in combination with a pharmaceutically acceptable carrier therefore.

8. A compound of Formula (I), as defined in any one of Claims 1 to 4 or a pharmaceutically acceptable salt thereof, for use in the treatment of obesity, hyperglycaemia, inflammation, or platelet aggregation in human or non-human animals.

## Revendications

1. Composé caractérisé en ce qu'il est représenté par la formule (I) ci-après:

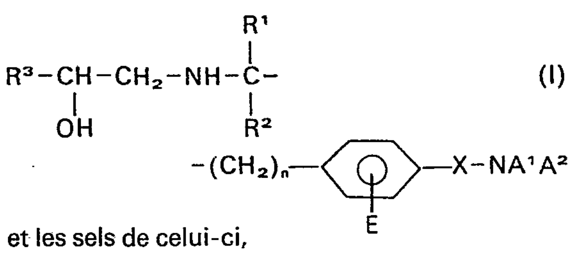

(I)

et les sels de celui-ci,

dans laquelle:

$R^1$ est un atome d'hydrogène ou un groupe méthyle;

$R^2$ est un atome d'hydrogène ou un groupe méthyle;

$R^3$ est un groupe phényle éventuellement substitué par un ou deux des radicaux suivants: un atome de fluor, de chlore, de brome, un groupe trifluorométhyle ou alcoyle en $C_{1-6}$; ou il est un groupe benzofuran-2-yle;

n est égal à 1 ou 2;

X est un radical alcoylène en $C_{1-15}$ à chaîne droite ou ramifiée;

$A^1$ est un atome d'hydrogène, un radical alcoyle en $C_{1-6}$ ou un groupe benzyle éventuellement substitué;

$A^2$ est un atome d'hydrogène, un groupe alcoyle en $C_{1-6}$, ou benzyle éventuellement substitué, et

E est un atome d'hydrogène, d'halogène, un groupe alcoxy en $C_{1-6}$, alcoyle en $C_{1-6}$ ou mono ou dialcoyl($C_{1-6}$)amino.

2. Composé suivant la revendication 1, caractérisé en ce que $R^3$ est un groupe phényle éventuellement substitué par l'un des radicaux suivants: un atome de fluor, de chlore, de brome ou le groupe trifluorométhyle; X est un groupe alcoylène en $C_{1-6}$ à chaîne droite ou ramifiée; $A^1$ est un atome d'hydrogène ou un groupe alcoyle en $C_{1-6}$; $A^2$ est un atome d'hydrogène ou un groupe alcoyle en $C_{1-6}$, et E est un atome d'hydrogène.

3. Composé suivant l'une des revendications 1 ou 2, caractérisé en ce que $A^1$ et $A^2$ sont simultanément un atome d'hydrogène ou bien l'un représente un atome d'hydrogène et l'autre un groupe méthyle.

4. Composé suivant l'une des revendications 1 à 3, caractérisé en ce que X représente le groupe méthylène.

5. Procédé de production d'un composé de formule (I), suivant la revendication 1, caractérisé en ce qu'il comprend la réduction d'un composé représenté par la formule (II) ci-après:

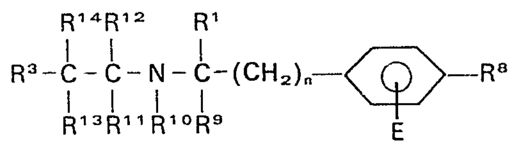

(II)

dans laquelle $R^1$, $R^3$, E et n sont tels que définis à propos de la formule (I), $R^8$ est un groupe $-X-NA^1A^2$ ou $-Y-Z-$ où $A^1$, $A^2$ et X sont tels que définis à propos de la formule (I) et Y est une liaison ou un groupe alcoylène en $C_{1-14}$ à chaîne droite ou ramifiée, et Z est un radical cyano ou

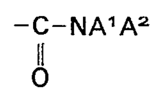

ou bien, Y est un groupe alcoylène en $C_{1-15}$ à chaîne droite ou ramifiée et Z est un radical

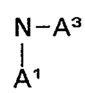

dans laquelle $A^3$ est un groupe alcoyl($C_{1-5}$)carbonyle, $R^9$ est un groupe $R^2$ tel que défini à propos de la formule (I) ou bien il forme une liaison avec $R^{10}$, $R^{10}$ est un atome d'hydrogène, un groupe benzyle ou, conjointement avec $R^9$ ou $R^{11}$, il forme une liaison; $R^{11}$ est un atome d'hydrogène ou, conjointement avec $R^{12}$, il forme un groupe oxo

ou, conjointement avec $R^{10}$, il forme une liaison; $R^{12}$ est un atome d'hydrogène où il forme un groupe oxo conjointement avec $R^{11}$; $R^{13}$ est un groupe hydroxyle ou forme un groupe oxo conjointement avec $R^{14}$; $R^{14}$ est un atome d'hydrogène ou forme un groupe oxo conjointement avec $R^{13}$, à condition qu'il n'y ait pas plus d'un groupe oxo et pas plus d'une liaison, représenté par l'un quelconque des $R^9$ à $R^{14}$ et, éventuellement ensuite, la formation d'un sel du composé de formule (I) ainsi formé en un autre composé de formule (I).

6. Procédé de production d'un composé de formule (I), caractérisé en ce qu'il comprend la réaction d'un composé représenté par la formule (III) ci-après:

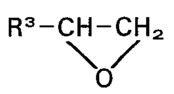 (III)

avec un composé représenté par la formule (IV) ci-après:

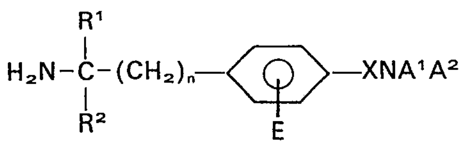 (IV)

dans lesquelles $R^1$, $R^2$, $R^3$, E, $A^1$, $A^2$, X et n sont tels que définis à propos de la formule (I) et, éventuellement ensuite, la formation d'un sel du composé de formule (I) ainsi formé et/ou la conversion du composé de formule (I) ainsi formé en un autre composé de formule (I).

7. Composition pharmaceutique caractérisée en ce qu'elle comprend un composé de formule (I) tel que défini suivant l'une des revendications 1 à 4, ou un sel acceptable du point de vue pharmaceutique de celui-ci, en association avec un support pour celui-ci, acceptable du point de vue pharmaceutique.

8. Composé de formule (I) suivant l'une des revendications 1 à 4, ou un sel de celui-ci acceptable du point de vue pharmaceutique, caractérisé en ce qu'il est utilisé dans le traitement de l'obésité, de l'hyperglycémie, de l'inflammation ou de l'agrégation des plaquettes chez l'être humain ou des animaux non humains.

**Patentansprüche**

1. Eine Verbindung der Formel (I):

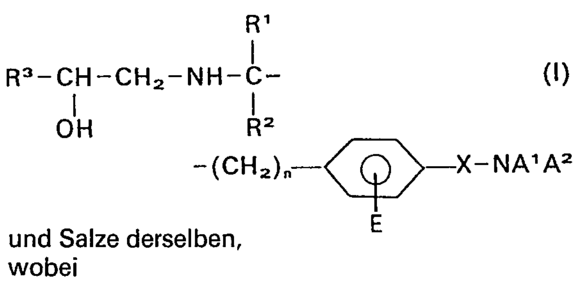 (I)

und Salze derselben,
wobei

$R^1$ Wasserstoff oder Methyl bedeutet;

$R^2$ Wasserstoff oder Methyl bedeutet;

$R^3$ Phenyl – gegebenenfalls substituiert mit einem oder zwei der folgenden Element bzw. Gruppen: Fluor, Chlor, Brom, Trifluoromethyl oder $C_{1-6}$-Alkyl – oder Benzfuran-2-yl ist;

n 1 oder 2 ist;

X ein geradkettiges oder verzweigtes $C_{1-15}$-Alkylen ist;

$A^1$ Wasserstoff, $C_{1-6}$-Alkyl, oder gegebenenfalls subtituiertes Benzyl ist;

$A^2$ Wasserstoff, $C_{1-6}$-Alkyl, oder gegebenenfalls subtituiertes Benzyl ist, und

E Wasserstoff, Halogen, $C_{1-6}$-Alkoxy, $C_{1-6}$-Alkyl oder Mono- oder Di-$C_{1-6}$-Alkylamin ist.

2. Eine Verbindung gemäss Anspruch 1, in welcher $R^3$ Phenyl, gegebenenfalls substituiert durch eines der folgenden Elemente bzw. eine der folgenden Gruppen: Fluor, Chlor, Brom oder Trifluormethyl;

X ein geradkettiges oder verzweigtes $C_{1-6}$-Alkylen ist;

$A^1$ Wasserstoff oder $C_{1-6}$-Alkyl ist; $A^2$ Wasserstoff oder $C_{1-6}$-Alkyl ist, und E Wasserstoff ist.

3. Eine Verbindung gemäss Anspruch 1 oder 2, in welcher $A^1$ und $A^2$ gleichzeitig Wasserstoff bedeuten, oder das eine Wasserstoff und das andere Methyl darstellt.

4. Eine Verbindung gemäss irgendeinem der Ansprüche 1 bis 3, in welcher X Methylen bedeutet.

5. Verfahren zur Herstellung einer Verbindung der Formel (I), wie in Anspruch 1 definiert, welches die Reduktion einer Verbindung der Formel (II):

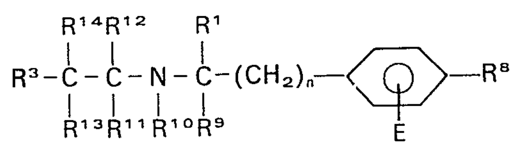 (II)

in welcher $R^1$, $R^3$, E und n wie in bezug auf Formel (I) definiert sind, $R^8$ eine Gruppe $-C-NA^1A^2$ oder $-Y-Z$ ist, wobei $A^1$ $A^2$ und X wie in bezug auf Formel (I) definiert sind und Y eine Bindung oder ein geradkettiges oder verzweigtes $C_{1-14}$-Alkylen ist und Z Cyano oder

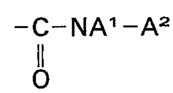

ist, oder Y ein geradkettiges oder verzweigtes Alkylen $C_{1-15}$-Alkylen ist und Z

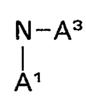

ist, wobei $A^3$ ein $C_{1-5}$-Alkylcarbonyl ist, $R^9$ eine Gruppe $R^2$, wie in bezug auf Formel (I) definiert, ist oder zusammen mit $R^{10}$ eine Bindung bildet; $R^{10}$ Wasserstoff oder Benzyl ist oder zusammen mit $R^9$ oder $R^{11}$ eine Bindung bildet; $R^{11}$ Wasserstoff ist oder zusammen mit $R^{12}$ eine Bindung bildet; $R^{12}$ Wasserstoff ist oder zusammen mit $R^{11}$ eine Oxo-Gruppe bildet; $R^{13}$ Hydroxyl ist oder zusammen mit $R^{14}$ eine Oxo-Gruppe bildet; $R^{14}$ Wasserstoff ist oder zusammen mit $R^{13}$ eine Oxo-

Gruppe bildet, vorausgesetzt, dass nicht mehr als eine Oxo-Gruppe und nicht mehr als eine Bindung durch irgendeine der Gruppen $R^9$ bis $R^{14}$ dargestellt werden und gegebenenfalls anschliessend die Bildung eines Salzes der so gebildeten Verbindung der Formel (I) und/oder Umwandlung der so gebildeten Verbindung der Formel (I) in eine weitere Verbindung der Formel (I), umfasst.

6. Ein Verfahren zur Herstellung einer Verbindung der Formel (I) wie in Anspruch 1 definiert, welches die Umsetzung einer Verbindung der Formel (III):

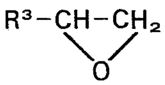

(III)

mit einer Verbindung der Formel (IV):

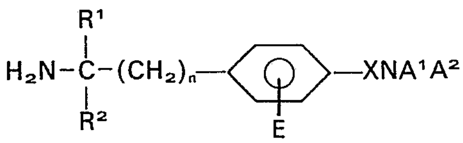

(IV)

in welcher $R^1$, $R^2$, $R^3$, E, $A^1$, $A^2$, X und n wie in bezug auf Formel (I) definiert sind, und gegebenenfalls anschliessend die Bildung eines Salzes der so gebildeten Verbindung der Formel (I) und/oder die Umwandlung der so gebildeten Verbindung der Formel (I) in eine weitere Verbindung der Formel (I) umfasst.

7. Eine pharmazeutische Zusammensetzung umfassend eine Verbindung der Formel (I), wie in irgendeinem der Ansprüche 1 bis 4 definiert, oder ein pharmazeutisch verträgliches Salz derselben, in Kombination mit einem pharmazeutisch verträglichen Träger dafür.

8. Eine Verbindung der Formel (I), wie in irgendeinem der Ansprüche 1 bis 4 beansprucht oder ein pharmazeutisch verträgliches Salz derselben, zur Verwendung bei der Behandlung von Fettsucht, Hyperglykämie, Entzündungen, oder Thrombozytenzusammenlagerungen bei Menschen oder Säugetieren.